# EUROPEAN PATENT APPLICATION

(11) **EP 4 613 845 A1**
(43) Date of publication of application: **10.09.2025**
(21) Application number: 23886202.3
(22) Date of filing: 31.10.2023
(51) Int. Cl.: C12N 5/00, C12N 15/85, C07K 16/00

(54) **METHOD FOR PREPARING HIGH-CONCENTRATION LIQUID MEDIUM**

(30) Priority: 31.10.2022 KR 20220143069
(71) Applicant: Samsung Bioepis Co., Ltd., Yeonsu-gu Incheon 21987 (KR)
(72) Inventor: KIM, Jin Woo, Incheon 21987 (KR); BONG, Ki Tae, Incheon 21987 (KR); LEE, Sang Min, Incheon 21987 (KR); LEE, Si Hyun, Incheon 21987 (KR); JANG, Hyun Yun, Incheon 21987 (KR); AHN, Hyeong Jun, Incheon 21987 (KR); SHIN, Jung Won, Incheon 21987 (KR); LEE, Seo Kyung, Incheon 21987 (KR); KIM, Kyoung Moon, Incheon 21987 (KR)
(74) Representative: Boult Wade Tennant LLP
(86) International application number: PCT/KR2023/017086
(87) International publication number: WO 2024/096506

(57) **Abstract**

The present disclosure relates to a method of preparing a high-concentration liquid medium having reduced osmolarity, and a method of producing a target protein using the medium.

A high-concentration liquid medium, when prepared according to one aspect, may have effectively reduced osmolarity. Therefore, the medium, when applied to a process for producing a target protein, may increase the production/expression level of the target protein, increase cell count, increase cell viability, and increase specific productivity of cells, thereby significantly increasing the production yield of the target protein from cells.

## Description

### Technical Field

The present disclosure relates to a method of preparing a high-concentration liquid medium having reduced osmotic pressure, and to a method of producing a target protein using the medium.

### Background Art

In the field of biotechnology, animal cell culture has been recognized as an important technology for the mass production of high-value-added materials, such as novel antibody production and vaccine development and manufacturing. Studying cell metabolism and regulatory mechanisms also plays a critical role in enhancing this economic value. Accordingly, extensive research has been conducted on medium components required for cell culture, and numerous efforts have been made to increase the productivity of desired proteins by adding various nutrients to the medium.

Recently, high-density cell seeding culture methods have been applied to animal cell culture for antibody production to increase the yield of recombinant proteins. When processes are carried out by inoculating cells at a high density, nutrients must be supplied through the medium in quantities proportional to the increased cell density to maintain cell viability and protein production capacity.

In the manufacture of such high-concentration media, conventional technology includes applying a high pH (approximately pH 8-9) to dissolve components that do not readily dissolve. To reach such a high pH, a large amount of base must be added, and to subsequently lower the manufactured medium back to neutral pH (pH 7.2), a large amount of acid must also be added. Consequently, using conventional technology results in significantly increased quantities of acid and base, which, in turn, causes a substantial increase in the osmotic pressure of the manufactured medium.

The osmotic pressure of the medium is one of the important factors influencing protein productivity in the culture process, and if the osmotic pressure of the medium exceeds the level that cells can tolerate, protein productivity may decrease. Therefore, there is a need to develop a method for minimizing the increase in osmotic pressure during the manufacture of high-concentration media.

### Disclosure of Invention

### Technical Problem

An aspect provides a method of preparing a high-concentration liquid medium, the method including: 1) mixing a first medium component with a solvent at a temperature of 35 °C or more to hydrate the first medium component; 2) cooling a solution containing the hydrated first medium component; and 3) mixing a second medium component with the solution to hydrate the second medium component.

Another aspect is to provide a high-concentration liquid medium prepared by the above method.

Another aspect is to provide a method of producing a target protein, the method including culturing a host cell expressing a target protein by using a high-concentration liquid medium prepared by the method described above.

### Solution to Problem

An aspect provides a method of preparing a high-concentration liquid medium, the method including: 1) mixing a first medium component with a solvent at a temperature of 35 °C or more to hydrate the first medium component; 2) cooling a solution containing the hydrated first medium component; and 3) mixing a second medium component with the solution to hydrate the second medium component. The same details described below equally apply to the above method.

In the present specification, the term "cell culture medium" refers to a solution containing the nutrients necessary for culturing host cells, specifically animal eukaryotic cells. In the present specification, the cell culture medium may be used interchangeably with "culture medium", "medium", or "growth medium". The medium may include commercially available or manufactured media used for culturing animal cells.

The solvent may be water (distilled water).

In the method, the solvent in step 1) may have a temperature of 35 °C to 100 °C, and specifically, the solvent may have a temperature of 35 °C to 100 °C, 35 °C to 90 °C, 35 °C to 80 °C, 35 °C to 75 °C, 35 °C to 70 °C, 35 °C to 65 °C, 35 °C to 60 °C, 35 °C to 55 °C, 35 °C to 50 °C, 35 °C to 45 °C, 35 °C to 40 °C, 40 °C to 100 °C, 40 °C to 90 °C, 40 °C to 80 °C, 40 °C to 75 °C, 40 °C to 70 °C, 40 °C to 65 °C, 40 °C to 60 °C, 40 °C to 55 °C, 40 °C to 50 °C, 40 °C to 45 °C, 45 °C to 100 °C, 45 °C to 90 °C, 45 °C to 80 °C, 45 °C to 75 °C, 45 °C to 70 °C, 45 °C to 65 °C, 45 °C to 60 °C, 50 °C to 100 °C, 50 °C to 90 °C, 50 °C to 80 °C, 50 °C to 75 °C, 50 °C to 70 °C, 50 °C to 65 °C, or 50 °C to 60 °C.

In the above method, the solvent may be maintained at a temperature of 35 °C to 100 °C while step 1) is carried out and specifically, the solvent may be maintained at a temperature of 35 °C to 100 °C while the first medium component is completely hydrated. The temperature at which the solvent is maintained may be 35 °C to 100 °C, 35 °C to 90 °C, 35 °C to 80 °C, 35 °C to 75 °C, 35 °C to 70 °C, 35 °C to 65 °C, 35 °C to 60 °C, 35 °C to 55 °C, 35 °C to 50 °C, 35 °C to 45 °C, 35 °C to 40 °C, 40 °C to 100 °C, 40 °C to 90 °C, 40 °C to 80 °C, 40 °C to 75 °C, 40 °C to 70 °C, 40 °C to 65 °C, 40 °C to 60 °C, 40 °C to 55 °C, 40 °C to 50 °C, 40 °C to 45 °C, 45 °C to 100 °C, 45 °C to 90 °C, 45 °C to 80 °C, 45 °C to 75 °C, 45 °C to 70 °C, 45 °C to 65 °C, 45 °C to 60 °C, 50 °C to 100 °C, 50 °C to 90 °C, 50 °C to 80 °C, 50 °C to 75 °C, 50 °C to 70 °C, 50 °C to 65 °C, or 50 °C to 60 °C.

The medium component may refer to a component included in an appropriate conventional medium used for cell culture and differentiation in the related art. The conventional medium may specifically be a basal medium or a cell culture minimum medium (CCMM), which generally includes a carbon source, a nitrogen source, and trace elements. Examples of the cell culture minimum medium may include one or more selected from the group consisting of DMEM (Dulbecco's Modified Eagle's Medium), MEM (Minimal Essential Medium), BME (Basal Medium Eagle), RPMI 1640, F-10, F-12, F-12K, DMEM/F12, α-MEM (α-Minimal Essential Medium), G-MEM (Glasgow's Minimal Essential Medium), IMDM (Iscove's Modified Dulbecco's Medium), McCoy's 5A medium, AmnioMax complete medium, AmnioMax ± medium, EBM (Endothelial Basal Medium), Chang's Medium, MesenCult-XF, DMEM/HG (Dulbecco's Modified Eagle's Medium high glucose), and MCDB+DMEM/LG (MCDB+Dulbecco's Modified Eagle's Medium low glucose). In addition, the conventional medium may be a chemically defined medium. In the present specification, the term "chemically defined medium" may refer to a growth medium suitable for in vitro culture of human or animal cells, in which all chemical components are known.

The medium component may include one or more selected from the group consisting of carbohydrates, amino acids, salts, minerals (trace elements), lipids, growth factors, vitamins, polyamines, nucleic acids, and buffers.

The carbohydrate may include any hexose such as, for example, glucose, galactose, fructose, or mannose, or a combination thereof.

The amino acid may include one or more selected from L-alanine, L-arginine, L-asparagine, L-aspartic acid, L-cysteine, cystine, L-ornithine, L-glutamic acid, L-glutamine, glycine, L-histidine, L-isoleucine, L-leucine, L-lysine, L-methionine, L-phenylalanine, L-proline, L-hydroxyproline, L-serine, L-threonine, L-tryptophan, L-tyrosine, and L-valine.

The salt may include, for example, one or more from Na⁺, K⁺, Mg²⁺, Ca²⁺, Cl⁻, SO₄²⁻, PO₄³⁻, HCO₃⁻, and salt forms thereof (for example, CaCl₂, KCl, NaCl, NaHCO₃, and Na₂HPO₄).

The mineral (trace element) may include, for example, one or more selected from Mn, Cu, Zn, Mo, Va, Se, Fe, Ca, Mg, Si, Ni, Al, Ag, Ba, Br, Cd, Co, Cr, F, Ge, J, Rb, Zr, and salt forms thereof (for example, CaCl₂, Fe(NO₃)₃, MgCl₂, MgSO₄, MnCl₂, NaCl, NaHCO₃, and Na₂HPO₄).

The vitamin may include, for example, one or more selected from biotin, choline chloride, folic acid, i-inositol, nicotinamide, D-Ca⁺⁺ pantothenate, pyridoxal, riboflavin, thiamine, pyridoxine, niacinamide, A, B6, B12, C, D3, E, K, and p-aminobenzoic acid (PABA).

The lipid may include, for example, one or more selected from fatty acids (for example, linoleic acid, linolenic acid, arachidonic acid, palmitoleic acid, oleic acid, polyenoic acids, and/or fatty acids having 12, 14, 16, 18, 20, or 24 carbon atoms, each carbon atom of which may be branched or unbranched), phospholipids, ethanolamine, lecithin (phosphatidylcholine), and cholesterol.

The buffer may include, for example, one or more selected from N-[2-hydroxyethyl] piperazine-N'-[2-ethanesulfonic acid] (HEPES), MOPS, MES, phosphates, bicarbonates, and other buffers suitable for use in cell culture applications.

The growth factor may include, for example, one or more selected from fibroblast growth factor (FGF) (including acidic FGF and basic FGF), insulin, insulin-like growth factor (IGF), epidermal growth factor (EGF), nerve growth factor (NGF), platelet-derived growth factor (PDGF), transforming growth factor (TGF) (including TGFα and TGFβ), and any cytokine (for example, interleukin 1, 2, 6, granulocyte-stimulating factor, and leukemia inhibitory factor (LIF)).

The polyamine may include, for example, one or more selected from putrescine, spermine, and spermidine.

The medium component may be classified into a first medium component and a second medium component.

The first medium component may include a component that does not undergo denaturation at a temperature of 35 °C or more. Specifically, the first medium component may include a component that does not undergo denaturation at a temperature of about 35 °C or more, about 40 °C or more, about 45 °C or more, about 50 °C or more, about 55 °C or more, about 60 °C or more, about 65 °C or more, about 70 °C or more, about 75 °C or more, about 80 °C or more, about 85 °C or more, about 90 °C or more, about 95 °C or more, or about 100 °C. More specifically, the first medium component may include a component that does not undergo denaturation at a temperature of about 35 °C or more to about 300 °C or less, about 35 °C or more to about 250 °C or less, about 35 °C or more to about 200 °C or less, about 35 °C or more to about 150 °C or less, about 35 °C or more to about 120 °C or less, about 40 °C or more to about 300 °C or less, about 40 °C or more to about 250 °C or less, about 40 °C or more to about 200 °C or less, about 40 °C or more to about 150 °C or less, about 40 °C or more to about 120 °C or less, about 45 °C or more to about 300 °C or less, about 45 °C or more to about 250 °C or less, about 45 °C or more to about 200 °C or less, about 45 °C or more to about 150 °C or less, about 45 °C or more to about 120 °C or less, about 50 °C or more to about 300 °C or less, about 50 °C or more to about 250 °C or less, about 50 °C or more to about 200 °C or less, about 50 °C or more to about 150 °C or less, about 50 °C or more to about 120 °C or less, about 55 °C or more to about 300 °C or less, about 55 °C or more to about 255 °C or less, about 55 °C or more to about 200 °C or less, about 55 °C or more to about 155 °C or less, about 55 °C or more to about 120 °C or less, about 60 °C or more to about 300 °C or less, about 60 °C or more to about 250 °C or less, about 60 °C or more to about 200 °C or less, about 60 °C or more to about 150 °C or less, about 60 °C or more to about 120 °C or less, about 70 °C or more to about 300 °C or less, about 70 °C or more to about 250 °C or less, about 70 °C or more to about 200 °C or less, about 70 °C or more to about 150 °C or less, about 70 °C or more to about 120 °C or less, about 80 °C or more to about 300 °C or less, about 80 °C or more to about 250 °C or less, about 80 °C or more to about 200 °C or less, about 80 °C or more to about 150 °C or less, about 80 °C or more to about 120 °C or less, about 90 °C or more to about 300 °C or less, about 90 °C or more to about 250 °C or less, about 90 °C or more to about 200 °C or less, about 90 °C or more to about 150 °C or less, or about 90 °C or more to about 120 °C or less.

In addition, the first medium component may not include a component capable of being denatured at a temperature of 35 °C or more. Specifically, the first medium component may not include a component capable of being denatured at about 35 °C or more, about 40 °C or more, about 45 °C or more, about 50 °C or more, about 55 °C or more, about 60 °C or more, about 65 °C or more, about 70 °C or more, about 75 °C or more, about 80 °C or more, about 85 °C or more, about 90 °C or more, about 95 °C or more, or about 100 °C. More specifically, the first medium component may not include a component capable of being denatured at about 35 °C or more to about 300 °C or less, about 35 °C or more to about 250 °C or less, about 35 °C or more to about 200 °C or less, about 35 °C or more to about 150 °C or less, about 35 °C or more to about 120 °C or less, about 40 °C or more to about 300 °C or less, about 40 °C or more to about 250 °C or less, about 40 °C or more to about 200 °C or less, about 40 °C or more to about 150 °C or less, about 40 °C or more to about 120 °C or less, about 45 °C or more to about 300 °C or less, about 45 °C or more to about 250 °C or less, about 45 °C or more to about 200 °C or less, about 45 °C or more to about 150 °C or less, about 45 °C or more to about 120 °C or less, about 50 °C or more to about 300 °C or less, about 50 °C or more to about 250 °C or less, about 50 °C or more to about 200 °C or less, about 50 °C or more to about 150 °C or less, about 50 °C or more to about 120 °C or less, about 55 °C or more to about 300 °C or less, about 55 °C or more to about 255 °C or less, about 55 °C or more to about 200 °C or less, about 55 °C or more to about 155 °C or less, about 55 °C or more to about 120 °C or less, about 60 °C or more to about 300 °C or less, about 60 °C or more to about 250 °C or less, about 60 °C or more to about 200 °C or less, about 60 °C or more to about 150 °C or less, about 60 °C or more to about 120 °C or less, about 70 °C or more to about 300 °C or less, about 70 °C or more to about 250 °C or less, about 70 °C or more to about 200 °C or less, about 70 °C or more to about 150 °C or less, about 70 °C or more to about 120 °C or less, about 80 °C or more to about 300 °C or less, about 80 °C or more to about 250 °C or less, about 80 °C or more to about 200 °C or less, about 80 °C or more to about 150 °C or less, about 80 °C or more to about 120 °C or less, about 90 °C or more to about 300 °C or less, about 90 °C or more to about 250 °C or less, about 90 °C or more to about 200 °C or less, about 90 °C or more to about 150 °C or less, or about 90 °C or more to about 120 °C or less.

In the present specification, the term "denaturation" may refer to a loss of the secondary, tertiary, or quaternary structures that exist in the native state of a protein or nucleic acid, resulting from the application of certain external stimuli or compounds such as strong acids or bases, heat, concentrated inorganic salts, organic solvents (alcohols or chloroform), or radiation. Specifically, the denaturation may mean that the original characteristics or functions are not maintained, including cases where biological activity is reduced or altered.

The first medium component may not include a protein or polypeptide. Specifically, the first medium component may not include a growth factor. More specifically, the first medium component may not include one or more selected from fibroblast growth factor (FGF) (including acidic FGF and basic FGF), insulin, insulin-like growth factor (IGF), epidermal growth factor (EGF), nerve growth factor (NGF), platelet-derived growth factor (PDGF), transforming growth factor (TGF) (including TGFα and TGFβ), and any cytokine (for example, interleukin 1, 2, 6, granulocyte-stimulating factor, and leukemia inhibitory factor (LIF)).

The hydration process may include dissolving (solubilizing) a solute (medium component) in a solvent.

In the method, the hydration process in step 1) may be performed until the first medium component is completely dissolved in the solvent, wherein this step may be carried out until the turbidity of the solution containing the dissolved first medium component is 10 NTU or less. Specifically, this step may be carried out until the turbidity of the solution is 10 NTU or less, 9 NTU or less, 8 NTU or less, 7 NTU or less, 6 NTU or less, 5 NTU or less, 4.5 NTU or less, 4 NTU or less, 3.5 NTU or less, 3 NTU or less, 2.5 NTU or less, 2 NTU or less, 1.5 NTU or less, 1 NTU or less, or 0.5 NTU or less. More specifically, this step may be performed until the turbidity of the solution reaches any value in the range of 0.1 NTU to 10 NTU, 0.1 NTU to 9 NTU, 0.1 NTU to 8 NTU, 0.1 NTU to 7 NTU, 0.1 NTU to 6 NTU, 0.1 NTU to 5 NTU, 0.1 NTU to 4.5 NTU, 0.1 NTU to 4 NTU, 0.1 NTU to 3.5 NTU, 0.1 NTU to 3 NTU, 0.1 NTU to 2.5 NTU, 0.1 NTU to 2 NTU, 0.1 NTU to 1.5 NTU, 0.1 NTU to 1 NTU, or 0.1 NTU to 0.5 NTU.

In the method, step 2) may include cooling the solution containing the first medium component hydrated therein, to a temperature of 40 °C or less, 35 °C or less, or 30 °C or less, and specifically to a temperature in a range of 10 °C to 30 °C. More specifically, the solution may be cooled to a temperature of 10 °C to 30 °C, 10 °C to 25 °C, 10 °C to 20 °C, 10 °C to 15 °C, 15 °C to 30 °C, 15 °C to 25 °C, 15 °C to 20 °C, 20 °C to 30 °C, 20 °C to 25 °C, or 25 °C to 30 °C, and more specifically, to room temperature.

The second medium component may include a component capable of being denatured at a temperature of 35 °C or more. Specifically, the second medium component may include a component capable of being denatured at a temperature of about 35 °C or more, about 40 °C or more, about 45 °C or more, about 50 °C or more, about 55 °C or more, about 60 °C or more, about 65 °C or more, about 70 °C or more, about 75 °C or more, about 80 °C or more, about 85 °C or more, about 90 °C or more, about 95 °C or more, or about 100 °C. More specifically, the second medium component may include a component capable of being denatured at a temperature of about 35 °C or more to about 300 °C or less, about 35 °C or more to about 250 °C or less, about 35 °C or more to about 200 °C or less, about 35 °C or more to about 150 °C or less, about 35 °C or more to about 120 °C or less, about 40 °C or more to about 300 °C or less, about 40 °C or more to about 250 °C or less, about 40 °C or more to about 200 °C or less, about 40 °C or more to about 150 °C or less, about 40 °C or more to about 120 °C or less, about 45 °C or more to about 300 °C or less, about 45 °C or more to about 250 °C or less, about 45 °C or more to about 200 °C or less, about 45 °C or more to about 150 °C or less, about 45 °C or more to about 120 °C or less, about 50 °C or more to about 300 °C or less, about 50 °C or more to about 250 °C or less, about 50 °C or more to about 200 °C or less, about 50 °C or more to about 150 °C or less, about 50 °C or more to about 120 °C or less, about 55 °C or more to about 300 °C or less, about 55 °C or more to about 255 °C or less, about 55 °C or more to about 200 °C or less, about 55 °C or more to about 155 °C or less, about 55 °C or more to about 120 °C or less, about 60 °C or more to about 300 °C or less, about 60 °C or more to about 250 °C or less, about 60 °C or more to about 200 °C or less, about 60 °C or more to about 150 °C or less, about 60 °C or more to about 120 °C or less, about 70 °C or more to about 300 °C or less, about 70 °C or more to about 250 °C or less, about 70 °C or more to about 200 °C or less, about 70 °C or more to about 150 °C or less, about 70 °C or more to about 120 °C or less, about 80 °C or more to about 300 °C or less, about 80 °C or more to about 250 °C or less, about 80 °C or more to about 200 °C or less, about 80 °C or more to about 150 °C or less, about 80 °C or more to about 120 °C or less, about 90 °C or more to about 300 °C or less, about 90 °C or more to about 250 °C or less, about 90 °C or more to about 200 °C or less, about 90 °C or more to about 150 °C or less, or about 90 °C or more to about 120 °C or less.

The second medium component may include a protein or polypeptide, and specifically may include a growth factor. More specifically, the second medium component may include one or more selected from fibroblast growth factor (FGF) (including acidic FGF and basic FGF), insulin, insulin-like growth factor (IGF), epidermal growth factor (EGF), nerve growth factor (NGF), platelet-derived growth factor (PDGF), transforming growth factor (TGF) (including TGFα and TGFβ), and any cytokine (for example, interleukin 1, 2, 6, granulocyte-stimulating factor, and leukemia inhibitory factor (LIF)).

In the above method, the hydration process in step 3) may be carried out until the second medium component is completely dissolved in the solvent, and this step may be performed until the turbidity of the solution containing the dissolved second medium component reaches 10 NTU or less. Specifically, this step may be performed until the turbidity of the solution containing the dissolved second medium component reaches 10 NTU or less, 9 NTU or less, 8 NTU or less, 7 NTU or less, 6 NTU or less, 5 NTU or less, 4.5 NTU or less, 4 NTU or less, 3.5 NTU or less, 3 NTU or less, 2.5 NTU or less, 2 NTU or less, 1.5 NTU or less, 1 NTU or less, 0.5 NTU or less. More specifically, this step may be performed until the turbidity of the solution containing the dissolved second medium component reaches any value in the range of 0.1 NTU to 10 NTU, 0.1 NTU to 9 NTU, 0.1 NTU to 8 NTU, 0.1 NTU to 7 NTU, 0.1 NTU to 6 NTU, 0.1 NTU to 5 NTU, 0.1 NTU to 4.5 NTU, 0.1 NTU to 4 NTU, 0.1 NTU to 3.5 NTU, 0.1 NTU to 3 NTU, 0.1 NTU to 2.5 NTU, 0.1 NTU to 2 NTU, 0.1 NTU to 1.5 NTU, 0.1 NTU to 1 NTU, or 0.1 NTU to 0.5 NTU.

In the above method, the medium components may not include sodium chloride, and specifically, both the first medium component and the second medium component may not include sodium chloride.

The method may further include step 4) of mixing an acid or a base with the solution containing the hydrated second medium component to adjust pH. The acid or base may refer to any suitable conventional acid or base used in the relevant art for adjusting the pH of a solution.

The step 4) may involve adjusting the pH of the solution to between 6.5 and 7.5, specifically, between 6.5 and 7.5, 6.5 and 7.3, 6.5 and 7.2, 6.5 and 7.1, 6.5 and 6.9, 6.5 and 6.7, 6.7 and 7.5, 6.7 and 7.3, 6.7 and 7.2, 6.7 and 7.1, 6.7 and 6.9, 6.9 and 7.5, 6.9 and 7.3, 6.9 and 7.2, 6.9 and 7.1, 7.1 and 7.5, 7.1 and 7.3, 7.1 and 7.2, 7.2 and 7.5, or 7.2 and 7.3, and more specifically, about 7.2.

The method may further include step 5) of additionally adding solvent to the solution to adjust the final concentration of the liquid medium.

The method may not include adjusting the pH with an acid or a base during the process of hydrating the first medium component or the second medium component. The method may not include adjusting the pH to 7.3 or higher with an acid or a base during the hydration process, and specifically, may not include adjusting the pH to 7.3 or higher, 7.5 or higher, 8.0 or higher, 8.5 or higher, 9.0 or higher, and more specifically, may not include adjusting the pH to between 7.3 and 13, 7.3 and 12, 7.3 and 11, 7.3 and 10, 7.3 and 9.5, 7.3 and 9, 7.3 and 8.5, 7.3 and 8, 7.5 and 13, 7.5 and 12, 7.5 and 11, 7.5 and 10, 7.5 and 9.5, 7.5 and 9, 7.5 and 8.5, 7.5 and 8, 8 and 13, 8 and 12, 8 and 11, 8 and 10, 8 and 9.5, 8 and 9, or 8 and 8.5.

In the above method, if the temperature of the solvent used in step 1) is in a range of 35 °C to 45 °C, the process of hydrating the first medium component and/or the second medium component may include adjusting the pH with an acid or a base. The adjustment of the pH may involve adjusting the pH to between 7.5 and 9.0 using a base, and specifically, may involve adjusting the pH to between 7.5 and 9.0, 7.5 and 8.8, 7.5 and 8.6, 7.5 and 8.5, 7.7 and 9.0, 7.7 and 8.8, 7.7 and 8.6, 7.7 and 8.5, 7.9 and 9.0, 7.9 and 8.8, 7.9 and 8.6, 7.9 and 8.5, 8.0 and 9.0, 8.0 and 8.8, 8.0 and 8.6, or 8.0 and 8.5.

The method may reduce the osmolarity of a high-concentration liquid medium, and specifically, may be a method of preparing a high-concentration liquid medium with reduced osmolarity.

The liquid medium prepared by the above method may have a reduced osmolarity compared to that of a liquid medium prepared by a method that uses the same medium components, and includes ① step of adjusting the pH to hydrate the medium components, and/or ② step of hydrating the medium components by using a solvent at 35 °C or less.

Specifically, in step ① in which the pH is adjusted to hydrate the medium components, the pH may be adjusted to 7.5 or higher, 8.0 or higher, 8.5 or higher, 9.0 or higher, between 7.5 and 13, between 7.5 and 12, between 7.5 and 11, between 7.5 and 10, between 7.5 and 9.5, between 7.5 and 9.0, between 7.5 and 8.5, between 7.5 and 8.0, between 8.0 and 13, between 8.0 and 12, between 8.0 and 11, between 8.0 and 10, between 8.0 and 9.5, between 8.0 and 9.0, between 8.0 and 8.5, between 8.5 and 13, between 8.5 and 12, between 8.5 and 11, between 8.5 and 10, between 8.5 and 9.5, between 8.5 and 9.0, between 9.0 and 13, between 9.0 and 12, between 9.0 and 11, between 9.0 and 10, or between 9.0 and 9.5.

In addition, in step ② in which the medium components are hydrated using a solvent at 35 °C or less, the solvent may be at 40 °C or less, 35 °C or less, 30 °C or less, 25 °C or less, 20 °C or less, 15 °C or less, 10 °C or less, 10 °C to 40 °C, 10 °C to 35 °C, 10 °C to 30 °C, 10 °C to 25 °C, 10 °C to 20 °C, 10 °C to 15 °C, 15 °C to 40 °C, 15 °C to 35 °C, 15 °C to 30 °C, 15 °C to 25 °C, 15 °C to 20 °C, 20 °C to 40 °C, 20 °C to 35 °C, 20 °C to 30 °C, 20 °C to 25 °C, 25 °C to 40 °C, 25 °C to 35 °C, 25 °C to 30 °C, 30 °C to 40 °C, 30 °C to 35 °C, or 30 °C to 40 °C.

The liquid medium prepared by the above method may have an osmolarity that is decreased by about 5 % or more compared to that of a liquid medium prepared by a method including a step of adjusting the pH to hydrate the medium components and/or a step of hydrating the medium components by using a solvent at 35 °C or less. More specifically, the decrease in osmolarity may be about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 5 % to about 30 %, about 5 % to about 20 %, about 5 % to about 10 %, about 10 % to about 60 %, about 10 % to about 50 %, about 10 % to about 40 %, about 10 % to about 30 %, about 10 % to about 20 %, about 15 % to about 60 %, about 15 % to about 50 %, about 15 % to about 40 %, about 15 % to about 30 %, about 15 % to about 20 %, about 20 % to about 60 %, about 20 % to about 50 %, about 20 % to about 40 %, about 20 % to about 30 %, about 30 % to about 60 %, about 30 % to about 50 %, about 30 % to about 40 %, about 40 % to about 60 %, about 40 % to about 50 %, about 50 % to about 60 %, about 60 % to about 70 %, about 70 % to about 80 %, about 80 % to about 90 %, or about 90 % to about 100 %. In addition, specifically, the decrease in osmolarity may be 5 % to 20 %, 5 % to 18 %, 5 % to 16 %, 5 % to 14 %, 5 % to 12 %, 5 % to 10 %, 7 % to 20 %, 7 % to 18 %, 7 % to 16 %, 7 % to 14 %, 7 % to 12 %, 7 % to 10 %, 9 % to 20 %, 9 % to 18 %, 9 % to 16 %, 9 % to 14 %, 9 % to 12 %, 9 % to 10 %, 11 % to 20 %, 11 % to 18 %, 11 % to 16 %, 11 % to 14 %, 11 % to 12 %, 13 % to 20 %, 13 % to 18 %, 13 % to 16 %, or 13 % to 14 %.

The liquid medium prepared by the above method may have an osmolarity of 500 mOsm/kg to 3,000 mOsm/kg, and more specifically, of 500 mOSm/kg to 3,000 mOsm/kg, 700 mOSm/kg to 3,000 mOsm/kg, 1,000 mOSm/kg to 3,000 mOsm/kg, 1,100 mOSm/kg to 3,000 mOsm/kg, 1,200 mOSm/kg to 3,000 mOsm/kg, 1,300 mOSm/kg to 3,000 mOsm/kg, 1,400 mOSm/kg to 3,000 mOsm/kg, 1,500 mOSm/kg to 3,000 mOsm/kg, 500 mOSm/kg to 2,500 mOsm/kg, 700 mOSm/kg to 2,500 mOsm/kg, 1,000 mOSm/kg to 2,500 mOsm/kg, 1,100 mOSm/kg to 2,500 mOsm/kg, 1,200 mOSm/kg to 2,500 mOsm/kg, 1,300 mOSm/kg to 2,500 mOsm/kg, 1,400 mOSm/kg to 2,500 mOsm/kg, 1,500 mOSm/kg to 2,500 mOsm/kg, 500 mOSm/kg to 2,000 mOsm/kg, 700 mOSm/kg to 2,000 mOsm/kg, 1,000 mOSm/kg to 2,000 mOsm/kg, 1,100 mOSm/kg to 2,000 mOsm/kg, 1,200 mOSm/kg to 2,000 mOsm/kg, 1,300 mOSm/kg to 2,000 mOsm/kg, 1,400 mOSm/kg to 2,000 mOsm/kg, 1,500 mOSm/kg to 2,000 mOsm/kg, 500 mOSm/kg to 1,500 mOsm/kg, 700 mOSm/kg to 1,500 mOsm/kg, 1,000 mOSm/kg to 1,500 mOsm/kg, 1,100 mOSm/kg to 1,500 mOsm/kg, 1,200 mOSm/kg to 1,500 mOsm/kg, 1,300 mOSm/kg to 1,500 mOsm/kg, or 1,400 mOSm/kg to 1,500 mOsm/kg.

The liquid medium prepared by the above method may contain total amino acids at a concentration of about 300 mM to about 1,500 mM, and specifically, may contain total amino acids at a concentration of about 300 mM to about 1,500 mM, 300 mM to 1,200 mM, 500 mM to 1,500 mM, 500 mM to 1,200 mM, 500 mM to 1,000 mM, 500 mM to 900 mM, 500 mM to 800 mM, 500 mM to 750 mM, 600 mM to 1,500 mM, 600 mM to 1,200 mM, 600 mM to 1,000 mM, 600 mM to 900 mM, 600 mM to 800 mM, 600 mM to 750 mM, 650 mM to 1,500 mM, 650 mM to 1,200 mM, 650 mM to 1,000 mM, 650 mM to 900 mM, 650 mM to 800 mM, 650 mM to 750 mM, 700 mM to 1,500 mM, 700 mM to 1,200 mM, 700 mM to 1,000 mM, 700 mM to 900 mM, 700 mM to 800 mM, or 700 mM to 750 mM.

The liquid medium prepared by the above method may exhibit an osmolality (mOsm/kg·mM) relative to total amino acid concentration of 0.5 mOsm/kg·mM to 3.0 mOsm/kg·mM, and specifically, may exhibit an osmolality (mOsm/kg·mM) relative to total amino acid concentration of 0.5 mOsm/kg·mM to 3.0 mOsm/kg·mM, 1.0 mOsm/kg·mM to 3.0 mOsm/kg·mM, 1.5 mOsm/kg·mM to 3.0 mOsm/kg·mM, 1.7 mOsm/kg·mM to 3.0 mOsm/kg·mM, 1.9 mOsm/kg·mM to 3.0 mOsm/kg·mM, 2.0 mOsm/kg·mM to 3.0 mOsm/kg·mM, 0.5 mOsm/kg·mM to 2.7 mOsm/kg·mM, 1.0 mOsm/kg·mM to 2.7 mOsm/kg·mM, 1.5 mOsm/kg·mM to 2.7 mOsm/kg·mM, 1.7 mOsm/kg·mM to 2.7 mOsm/kg·mM, 1.9 mOsm/kg·mM to 2.7 mOsm/kg·mM, 2.0 mOsm/kg·mM to 2.7 mOsm/kg·mM, 0.5 mOsm/kg·mM to 2.5 mOsm/kg·mM, 1.0 mOsm/kg·mM to 2.5 mOsm/kg·mM, 1.5 mOsm/kg·mM to 2.5 mOsm/kg·mM, 1.7 mOsm/kg·mM to 2.5 mOsm/kg·mM, 1.9 mOsm/kg·mM to 2.5 mOsm/kg·mM, 2.0 mOsm/kg·mM to 2.5 mOsm/kg·mM, 0.5 mOsm/kg·mM to 2.3 mOsm/kg·mM, 1.0 mOsm/kg·mM to 2.3 mOsm/kg·mM, 1.5 mOsm/kg·mM to 2.3 mOsm/kg·mM, 1.7 mOsm/kg·mM to 2.3 mOsm/kg·mM, 1.9 mOsm/kg·mM to 2.3 mOsm/kg·mM, 2.0 mOsm/kg·mM to 2.3 mOsm/kg·mM, 0.5 mOsm/kg·mM to 2.1 mOsm/kg·mM, 1.0 mOsm/kg·mM to 2.1 mOsm/kg·mM, 1.5 mOsm/kg·mM to 2.1 mOsm/kg·mM, 1.7 mOsm/kg·mM to 2.1 mOsm/kg·mM, 1.9 mOsm/kg·mM to 2.1 mOsm/kg·mM, or 2.0 mOsm/kg·mM to 2.1 mOsm/kg·mM.

The liquid medium prepared by the above method may exhibit an osmolality (mOsm/kg·mM) relative to total amino acid concentration that is decreased by about 5 % or more compared to that of a liquid medium prepared by a method including a step of adjusting the pH to hydrate the medium components and/or a step of hydrating the medium components by using a solvent at 35 °C or less. Specifically, the decrease in osmolality (mOsm/kg·mM) relative to total amino acid concentration may be about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 5 % to about 30 %, about 5 % to about 20 %, about 5 % to about 10 %, about 10 % to about 60 %, about 10 % to about 50 %, about 10 % to about 40 %, about 10 % to about 30 %, about 10 % to about 20 %, about 15 % to about 60 %, about 15 % to about 50 %, about 15 % to about 40 %, about 15 % to about 30 %, about 15 % to about 20 %, about 20 % to about 60 %, about 20 % to about 50 %, about 20 % to about 40 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 %.

The above method may be for preparing a high-concentration liquid medium with improved target protein productivity. Specifically, the liquid medium prepared by this method may provide improved target protein productivity compared to a liquid medium prepared by a method including the step of adjusting the pH to hydrate the medium components and/or hydrating the medium components by using a solvent at 35 °C or less.

The improved target protein productivity may include one or more of the following characteristics:
1) improved viability of host cells that express or produce a target protein; 2) improved specific productivity (productivity per unit cell, qp) of the host cells; 3) improved viable cell density (VCD) of the host cells in a target protein production process; and 4) improved expression/production level (titer) of a target protein in a target protein production process.

In the present specification, the terms "target protein expression level in cells" and "titer" may be used interchangeably. The target protein expression level in the cells may be expressed as the concentration (g/L) of the protein in the cell culture medium obtained through the process.

The cell viability refers to the ability of cultured cells to survive, grow, or proliferate in the culture environment, and cell viability (%) may be calculated as the relative ratio of live cells in the experimental group compared to a negative control group.

When a liquid medium prepared by the above method is used in a target protein production process, the expression/production level (titer) of the target protein in such a process may be increased compared to the expression/production level achieved using a liquid medium prepared by a method including a step of adjusting the pH to hydrate the medium components and/or a step of hydrating the medium components by using a solvent at 35 °C or less, and specifically, the expression/production level (titer) of the target protein in the target protein production process may be increased by about 5 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 10 % to about 60 %, about 10 % to about 50 %, about 10 % to about 40 %, about 15 % to about 60 %, about 15 % to about 50 %, about 15 % to about 40 %, about 15 % to about 30 %, about 15 % to about 20 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 % compared to the expression/production level achieved using a liquid medium prepared by a method including a step of adjusting the pH to hydrate the medium components and/or a step of hydrating the medium components by using a solvent at 35 °C or less.

When a liquid medium prepared by the above method is used in a target protein production process, the cell viability in such a process may be increased compared to the viability achieved using a liquid medium prepared by a method including a step of adjusting the pH to hydrate the medium components and/or a step of hydrating the medium components by using a solvent at or below 35 °C, and specifically, the cell viability in the target protein production process may be increased by about 3 % or more, about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 3 % to about 50 %, about 3 % to about 40 %, about 3 % to about 30 %, about 3 % to about 20 %, about 3 % to about 10 %, about 3 % to about 5 %, about 5 % to about 50 %, about 5 % to about 40 %, about 5 % to about 30 %, about 5 % to about 20 %, about 5 % to about 10 %, about 10 % to about 15 %, about 15 % to about 20 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 % compared to the viability achieved using a liquid medium prepared by a method including a step of adjusting the pH to hydrate the medium components and/or a step of hydrating the medium components by using a solvent at or below 35 °C.

When a liquid medium prepared by the above method is used in a target protein production process, the viable cell density (VCD) in such a process may be increased compared to the VCD achieved using a liquid medium prepared by a method including a step of adjusting the pH to hydrate the medium components and/or a step of hydrating the medium components by using a solvent at 35 °C or less, and specifically, the viable cell density (VCD) in the target protein production process may be increased by about 5 % or more, about 10 % or more, about 15 % or more, about 20 % or more, about 30 % or more, about 40 % or more, about 50 % or more, about 60 % or more, about 70 % or more, about 80 % or more, about 90 % or more, about 3 % to 5 %, about 5 % to about 10 %, about 5 % to about 60 %, about 5 % to about 50 %, about 5 % to about 40 %, about 5 % to about 30 %, about 5 % to about 20 %, about 10 % to about 60 %, about 10 % to about 50 %, about 10 % to about 40 %, about 10 % to about 30 %, about 10 % to about 20 %, about 10 % to about 15 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 % compared to the VCD achieved using a liquid medium prepared by a method including a step of adjusting the pH to hydrate the medium components and/or a step of hydrating the medium components by using a solvent at 35 °C or less.

When a liquid medium prepared by the above method is used in a target protein production process, the specific productivity (productivity per unit cell, qp) of host cells in SSsuch a process may be increased compared to the specific productivity achieved using a liquid medium prepared by a method including a step of adjusting the pH to hydrate the medium components and/or a step of hydrating the medium components by using a solvent at 35 °C or less, and specifically, the specific productivity (qp) of host cells in the target protein production process may be increased by about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 10 % to about 60 %, about 10 % to about 50 %, about 10 % to about 40 %, about 15 % to about 60 %, about 15 % to about 50 %, about 15 % to about 40 %, about 15 % to about 30 %, about 15 % to about 20 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 % compared to the specific productivity achieved using a liquid medium prepared by a method including a step of adjusting the pH to hydrate the medium components and/or a step of hydrating the medium components by using a solvent at 35 °C or less.

In an embodiment, the method may be a method of preparing a high-concentration liquid medium, the method including: 1) mixing a first medium component with a solvent at a temperature of 35 °C to 45 °C to hydrate the first medium component; 2) cooling a solution containing the hydrated first medium component to room temperature; 3) mixing a second medium component with the solution to hydrate the second medium component; and 4) adjusting the pH of the solution to between 7.0 and 7.5, wherein step 1) and/or step 3) includes adjusting the pH of the solvent/solution to between 7.5 and 8.5 to hydrate the medium components, wherein the liquid medium prepared by this method has an osmolality that is decreased by about 10 % or more compared to that of a liquid medium prepared by a method including a step of adjusting the pH to 8.5 or higher to hydrate the medium components and/or a step of hydrating the medium components by using a solvent at 35 °C or less.

In an embodiment, the method may be a method of preparing a high-concentration liquid medium, the method including: 1) mixing a first medium component with a solvent at a temperature of 45 °C to 65 °C to hydrate the first medium component; 2) cooling a solution containing the hydrated first medium component to room temperature; 3) mixing a second medium component with the solution to hydrate the second medium component; and 4) adjusting the pH of the solution to between 7.0 and 7.5, wherein the liquid medium prepared by this method has an osmolality that is decreased by about 10% or more compared to that of a liquid medium prepared by a method including a step of adjusting the pH to 8.5 or higher to hydrate the medium components and/or a step of hydrating the medium components by using a solvent at 35 °C or less.

Another aspect is to provide a high-concentration liquid medium or medium composition prepared by the above method. The same details as described above equally apply to the medium or medium composition.

The high-concentration liquid medium may have decreased osmolality, and specifically, the osmolality of the high-concentration liquid medium may be decreased compared to that of a liquid medium prepared by a method that includes: ① step of adjusting the pH to hydrate the medium components; and/or ② step of hydrating the medium components by using a solvent at 35 °C or less.

In step ① in which the pH is adjusted to hydrate the medium components, the pH may be adjusted to 7.5 or higher, 8.0 or higher, 8.5 or higher, 9.0 or higher, between 7.5 and 13, between 7.5 and 12, between 7.5 and 11, between 7.5 and 10, between 7.5 and 9.5, between 7.5 and 9.0, between 7.5 and 8.5, between 7.5 and 8.0, between 8.0 and 13, between 8.0 and 12, between 8.0 and 11, between 8.0 and 10, between 8.0 and 9.5, between 8.0 and 9.0, between 8.0 and 8.5, between 8.5 and 13, between 8.5 and 12, between 8.5 and 11, between 8.5 and 10, between 8.5 and 9.5, between 8.5 and 9.0, between 9.0 and 13, between 9.0 and 12, between 9.0 and 11, between 9.0 and 10, or between 9.0 and 9.5.

In addition, in step ② in which the medium components are hydrated using a solvent at 35 °C or less, the solvent may be at a temperature of 35 °C or less, 30 °C or less, 25 °C or less, 20 °C or less, 15 °C or less, 10 °C or less, 10 °C to 40 °C, 10 °C to 35 °C, 10 °C to 30 °C, 10 °C to 25 °C, 10 °C to 20 °C, 10 °C to 15 °C, 15 °C to 40 °C, 15 °C to 35 °C, 15 °C to 30 °C, 15 °C to 25 °C, 15 °C to 20 °C, 20 °C to 40 °C, 20 °C to 35 °C, 20 °C to 30 °C, 20 °C to 25 °C, 25 °C to 40 °C, 25 °C to 35 °C, 25 °C to 30 °C, 30 °C to 40 °C, 30 °C to 35 °C, or 30 °C to 40 °C.

The high-concentration liquid medium may have an osmolality decreased by about 5 % compared to that of a liquid medium prepared by a method including a step of adjusting the pH to hydrate the medium components and/or a step of hydrating the medium components by using a solvent at 35 °C or less. Specifically, the decrease in osmolality may be about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 5 % to about 30 %, about 5 % to about 20 %, about 5 % to about 10 %, about 10 % to about 60 %, about 10 % to about 50 %, about 10 % to about 40 %, about 10 % to about 30 %, about 10 % to about 20 %, about 15 % to about 60 %, about 15 % to about 50 %, about 15 % to about 40 %, about 15 % to about 30 %, about 15 % to about 20 %, about 20 % to about 60 %, about 20 % to about 50 %, about 20 % to about 40 %, about 20 % to about 30 %, about 30 % to about 60 %, about 30 % to about 50 %, about 30 % to about 40 %, about 40 % to about 60 %, about 40 % to about 50 %, about 50 % to about 60 %, about 60 % to about 70 %, about 70 % to about 80 %, about 80 % to about 90 %, or about 90 % to about 100 %. In addition, specifically, the decrease in osmolality may be 5 % to 20 %, 5 % to 18 %, 5 % to 16 %, 5 % to 14 %, 5 % to 12 %, 5 % to 10 %, 7 % to 20 %, 7 % to 18 %, 7 % to 16 %, 7 % to 14 %, 7 % to 12 %, 7 % to 10 %, 9 % to 20 %, 9 % to 18 %, 9 % to 16 %, 9 % to 14 %, 9 % to 12 %, 9 % to 10 %, 11 % to 20 %, 11 % to 18 %, 11 % to 16 %, 11 % to 14 %, 11 % to 12 %, 13 % to 20 %, 13 % to 18 %, 13 % to 16 %, or 13 % to 14 %.

The high-concentration liquid medium may have an osmolality of 500 mOsm/kg to 3,000 mOsm/kg, and specifically, may have an osmolality of 500 mOsm/kg to 3,000 mOsm/kg, 700 mOsm/kg to 3,000 mOsm/kg, 1,000 mOsm/kg to 3,000 mOsm/kg, 1,100 mOsm/kg to 3,000 mOsm/kg, 1,200 mOsm/kg to 3,000 mOsm/kg, 1,300 mOsm/kg to 3,000 mOsm/kg, 1,400 mOsm/kg to 3,000 mOsm/kg, 1,500 mOsm/kg to 3,000 mOsm/kg, 500 mOsm/kg to 2,500 mOsm/kg, 700 mOsm/kg to 2,500 mOsm/kg, 1,000 mOsm/kg to 2,500 mOsm/kg, 1,100 mOsm/kg to 2,500 mOsm/kg, 1,200 mOsm/kg to 2,500 mOsm/kg, 1,300 mOsm/kg to 2,500 mOsm/kg, 1,400 mOsm/kg to 2,500 mOsm/kg, 1,500 mOsm/kg to 2,500 mOsm/kg, 500 mOsm/kg to 2,000 mOsm/kg, 700 mOsm/kg to 2,000 mOsm/kg, 1,000 mOsm/kg to 2,000 mOsm/kg, 1,100 mOsm/kg to 2,000 mOsm/kg, 1,200 mOsm/kg to 2,000 mOsm/kg, 1,300 mOsm/kg to 2,000 mOsm/kg, 1,400 mOsm/kg to 2,000 mOsm/kg, 1,500 mOsm/kg to 2,000 mOsm/kg, 500 mOsm/kg to 1,500 mOsm/kg, 700 mOsm/kg to 1,500 mOsm/kg, 1,000 mOsm/kg to 1,500 mOsm/kg, 1,100 mOsm/kg to 1,500 mOsm/kg, 1,200 mOsm/kg to 1,500 mOsm/kg, 1,300 mOsm/kg to 1,500 mOsm/kg, or 1,400 mOsm/kg to 1,500 mOsm/kg. The high-concentration liquid medium may include total amino acids at a concentration of 300 mM to 1,500 mM, and specifically, may include total amino acids at a concentration of 300 mM to 1,500 mM, 300 mM to 1,200 mM, 500 mM to 1,500 mM, 500 mM to 1,200 mM, 500 mM to 1,000 mM, 500 mM to 900 mM, 500 mM to 800 mM, 500 mM to 750 mM, 600 mM to 1,500 mM, 600 mM to 1,200 mM, 600 mM to 1,000 mM, 600 mM to 900 mM, 600 mM to 800 mM, 600 mM to 750 mM, 650 mM to 1,500 mM, 650 mM to 1,200 mM, 650 mM to 1,000 mM, 650 mM to 900 mM, 650 mM to 800 mM, 650 mM to 750 mM, 700 mM to 1,500 mM, 700 mM to 1,200 mM, 700 mM to 1,000 mM, 700 mM to 900 mM, 700 mM to 800 mM, or 700 mM to 750 mM.

The high-concentration liquid medium may have an osmolality (mOsm/kg·mM) relative to total amino acid concentration of 0.5 mOsm/kg·mM to 3.0 mOsm/kg·mM, and specifically, may exhibit an osmolality (mOsm/kg·mM) relative to total amino acid concentration of 0.5 mOsm/kg·mM to 3.0 mOsm/kg·mM, 1.0 mOsm/kg·mM to 3.0 mOsm/kg·mM, 1.5 mOsm/kg·mM to 3.0 mOsm/kg·mM, 1.7 mOsm/kg·mM to 3.0 mOsm/kg·mM, 1.9 mOsm/kg·mM to 3.0 mOsm/kg·mM, 2.0 mOsm/kg·mM to 3.0 mOsm/kg·mM, 0.5 mOsm/kg·mM to 2.7 mOsm/kg·mM, 1.0 mOsm/kg·mM to 2.7 mOsm/kg·mM, 1.5 mOsm/kg·mM to 2.7 mOsm/kg·mM, 1.7 mOsm/kg·mM to 2.7 mOsm/kg·mM, 1.9 mOsm/kg·mM to 2.7 mOsm/kg·mM, 2.0 mOsm/kg·mM to 2.7 mOsm/kg·mM, 0.5 mOsm/kg·mM to 2.5 mOsm/kg·mM, 1.0 mOsm/kg·mM to 2.5 mOsm/kg·mM, 1.5 mOsm/kg·mM to 2.5 mOsm/kg·mM, 1.7 mOsm/kg·mM to 2.5 mOsm/kg·mM, 1.9 mOsm/kg·mM to 2.5 mOsm/kg·mM, 2.0 mOsm/kg·mM to 2.5 mOsm/kg·mM, 0.5 mOsm/kg·mM to 2.3 mOsm/kg·mM, 1.0 mOsm/kg·mM to 2.3 mOsm/kg·mM, 1.5 mOsm/kg·mM to 2.3 mOsm/kg·mM, 1.7 mOsm/kg·mM to 2.3 mOsm/kg·mM, 1.9 mOsm/kg·mM to 2.3 mOsm/kg·mM, 2.0 mOsm/kg·mM to 2.3 mOsm/kg·mM, 0.5 mOsm/kg·mM to 2.1 mOsm/kg·mM, 1.0 mOsm/kg·mM to 2.1 mOsm/kg·mM, 1.5 mOsm/kg·mM to 2.1 mOsm/kg·mM, 1.7 mOsm/kg·mM to 2.1 mOsm/kg·mM, 1.9 mOsm/kg·mM to 2.1 mOsm/kg·mM, or 2.0 mOsm/kg·mM to 2.1 mOsm/kg·mM.

The high-concentration liquid medium may exhibit an osmolality (mOsm/kg·mM) relative to total amino acid concentration that is decreased by 5 % or more compared to that of a liquid medium prepared by a method including a step of adjusting the pH to hydrate the medium components and/or a step of hydrating the medium components by using a solvent at 35 °C or less. Specifically, the decrease in the osmolality relative to total amino acid concentration may be about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 5 % to about 30 %, about 5 % to about 20 %, about 5 % to about 10 %, about 10 % to about 60 %, about 10 % to about 50 %, about 10 % to about 40 %, about 10 % to about 30 %, about 10 % to about 20 %, about 15 % to about 60 %, about 15 % to about 50 %, about 15 % to about 40 %, about 15 % to about 30 %, about 15 % to about 20 %, about 20 % to about 60 %, about 20 % to about 50 %, about 20 % to about 40 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 %.

Another aspect is to provide a method of producing a target protein, the method including culturing host cells that express a target protein by using the high-concentration liquid medium prepared by the above method. The same details as described above equally apply to the above method.

In the present specification, the term "target protein" refers to any protein that a person skilled in the art intends to express or obtain, including any protein capable of being expressed in a host cell or in a desired organism. In the present specification, the target protein may be used interchangeably with "target protein" or "protein of interest".

The target protein may be a recombinant protein and for example, may include those used as antibody therapeutics, anti-cancer immunotherapeutics, cancer treatment vaccines, infectious disease vaccines, protein replacement therapeutics, allergy therapeutics, or immunotherapeutics for immune-related diseases.

The target protein may be selected from the group consisting of: antibodies or antigen-binding fragments thereof, immunoadhesins, Transforming Growth Factor (TGF)-beta superfamily signaling molecules, blood coagulation factors, anti-tumor necrosis factor receptor (TNFR) antibodies, anti-human epidermal growth factor receptor 2 (HER2) antibodies, and TNFR:Fc.

In the present specification, the term "antibody" is used interchangeably with the term "immunoglobulin (Ig)". A complete antibody has two full-length light chains and two full-length heavy chains, and each light chain is bound to a heavy chain via a disulfide bond (SS-bond). The antibody may be, for example, IgA, IgD, IgE, IgG, or IgM. The antibody may be a monoclonal antibody or a polyclonal antibody. The antibody may be an animal-derived antibody, a mouse-human chimeric antibody, a humanized antibody, or a human antibody. As used herein, the term "antigen-binding fragment" refers to a fragment of the complete immunoglobulin structure, specifically referring to a portion of the polypeptide that contains the antigen-binding region. For example, the antigen-binding fragment may be scFv, (scFv)₂, Fv, Fab, Fab', Fv F(ab')₂, or a combination thereof.

The immunoadhesin is an antibody-like protein, and refers to a fusion protein of an immunoglobulin Fc region and a functional domain of a binding protein (e.g., a receptor, ligand, or cell-adhesion molecule).

The TGF-beta superfamily signaling molecule may be, for example, TGF-beta 1, TGF-beta 2, or TGF-beta 3. The blood coagulation factor refers to a factor involved in blood coagulation. The blood coagulation factor may be, for example, fibrinogen, prothrombin, tissue thromboplastin, Factor V, Factor VII, Factor VIII, Factor IX, Factor X, Factor XI, Factor XII, Factor XIII, von Willebrand factor, prekallikrein, high-molecular-weight kininogen, fibronectin, antithrombin III, heparin cofactor II, Protein C, Protein S, Protein Z, Protein Z-dependent protease inhibitor, plasminogen, alpha 2-antiplasmin, tissue plasminogen activator, urokinase, plasminogen activator inhibitor-1, plasminogen activator inhibitor-2, or cancer procoagulant.

The anti-TNFR antibody may be an antibody that specifically binds to TNFR or an antigen-binding fragment thereof. In addition, the anti-HER2 antibody may be an antibody that specifically binds to HER2 or an antigen-binding fragment thereof, and "TNFR:Fc" refers to a fusion protein of TNFR and an Fc region.

The target protein may be selected from the group consisting of abagovomab, abciximab, adalimumab, adecatumumab, alemtuzumab, altumomab, altumomab pentetate, anatumomab, anatumomab mafenatox, arcitumomab, atlizumab, basiliximab, bectumomab, ectumomab, belimumab, benralizumab, bevacizumab, brentuximab, canakinumab, capromab, capromab pendetide, catumaxomab, certolizumab, clivatuzumab tetraxetan, daclizumab, denosumab, eculizumab, edrecolomab, efalizumab, etaracizumab, ertumaxomab, fanolesomab, fontolizumab, gemtuzumab, girentuximab, golimumab, ibritumomab, igovomab, infliximab, ipilimumab, labetuzumab, mepolizumab, muromonab, muromonab-CD3, natalizumab, necitumumab, nimotuzumab, ofatumumab, omalizumab, oregovomab, palivizumab, panitumumab, ranibizumab, rituximab, satumomab, sulesomab, ibritumomab, ibritumomab tiuxetan, tocilizumab, tositumomab, trastuzumab, ustekinumab, visilizumab, votumumab, zalutumumab, brodalumab, anrukinzumab, bapineuzumab, dalotuzumab, demcizumab, ganitumab, inotuzumab, mavrilimumab, moxetumomab pasudotox, rilotumumab, sifalimumab, tanezumab, tralokinumab, tremelimumab, urelumab, adornase alfa, REBIF, becaplermin, alteplase, laronidase, alefacept, aflibercept, raxibacumab, darbepoetin alfa, becaplermin concentrate, interferon beta-1b, Botulinum toxin type A, rasburicase, asparaginase, epoetin alfa, etanercept, agalsidase beta, interferon alfacon-1, interferon alfa-2a, anakinra, Botulinum toxin type B, pegfilgrastim, oprelvekin, filgrastim, denileukin diftitox, peginterferon alfa-2a, aldesleukin, dornase alfa, interferon beta-1a, becaplermin, reteplase, interferon alfa-2, tenecteplase, drotrecogin alfa, rilonacept, romiplostim, methoxypolyethylene glycol-epoetin beta, C1 esterase inhibitor, idursulfase, alglucosidase alfa, abatacept, galsulfase, palifermin, and interferon gamma-1b.

The host cell or organism that expresses or produces the target protein may be any suitable host cell or organism capable of expressing the target protein, and may be transformed with a gene encoding the target protein. The host cell may be eukaryotic or prokaryotic, including, without limitation, bacterial cells, yeast cells, insect cells, and mammalian cells, with mammalian cells being preferred. The mammalian cells refer to cells derived from, for example, mouse, rat, rabbit, dog, cat, sheep, cow, horse, monkey, chimpanzee, or human. The cell may be a cell line.

The host cell may be selected from the group consisting of, for example, CHO (Chinese hamster ovary) cells, DG44 cells, HEK (human embryonic kidney) cells, NS0 cells, PER.C6 cells, HeLa cells, MDCK (Madin-Darby Canine Kidney) cells, COS7 (monkey kidney) cells, SP2/0 cells, W138 cells, BHK (baby hamster kidney) cells, myeloma cell lines, and HuT 78 cells. The CHO cell may be a CHO K1 or CHO DUKX cell. The HEK cell may be an HEK 293 cell.

The culturing step may include culturing the cells under conditions permitting their survival or proliferation.

The conditions permitting cell survival or proliferation may vary depending on the cell type. For example, the cells may be cultured in the presence of a medium containing the nutrients necessary for cell proliferation. The cells may be cultured at about 25 °C to about 42 °C, about 25 °C to about 40 °C, about 30 °C to about 40 °C, about 30 °C to about 37 °C, or about 37 °C. The cells may be cultured in the presence of approximately 1 % CO₂ to approximately 10 % CO₂, or approximately 5 % CO₂ to approximately 10 % CO₂.

The culturing process may vary depending on the cell type The culturing process may use any known method. The culturing process may be carried out in plates, flasks, incubators, or the like, The culturing process may be performed either by allowing the cells to adhere to a substrate or by suspending them in the culture medium. The culturing process may be performed by subculture (passage). The culturing process may be, for example, a batch culture, a fed-batch culture, a perfusion culture, or a continuous culture, but is not limited thereto. During culturing, the cell culture medium may be periodically replaced with fresh medium. The cells may be cultured for about 1 day or longer, about 2 days or longer, about 3 days or longer, about 4 days or longer, about 5 days or longer, about 6 days or longer, about 1 week or longer, about 10 days or longer, about 2 weeks or longer, about 3 weeks or longer, about 1 month or longer, about 1 day to about 1 month, about 1 day to about 3 weeks, about 1 day to about 2 weeks, about 2 days to about 2 weeks, about 3 days to about 2 weeks, about 4 days to about 2 weeks, about 5 days to about 2 weeks, about 6 days to about 2 weeks, or about 1 week to about 2 weeks.

The method may include at least one temperature change condition, at least one pH change condition, or a combination thereof.

Under the temperature change condition, the cells may grow in the medium at a first temperature for 3 days or longer, the temperature may then be changed to a second temperature that is about 1 °C to about 8 °C lower than the first temperature, and the cells may be maintained at the second temperature for about 2 days or longer. The cells may grow in the medium at the first temperature for about 3 days or longer, about 4 days or longer, about 1 week or longer, about 2 days to about 1 week, or about 2 days to about 4 days. The cells may be maintained in the medium at the second temperature for about 2 days or longer, about 3 days or longer, about 4 days or longer, about 1 week or longer, about 2 weeks or longer, about 3 weeks or longer, about 1 month or longer, about 1 day to about 1 month, about 1 day to about 3 weeks, about 1 day to about 2 weeks, about 1 day to about 1 week, or about 1 day to about 4 days. The first temperature may be about 30 °C to about 42 °C, about 32 °C to about 42 °C, about 32 °C to about 40 °C, about 34 °C to about 40 °C, about 36 °C to about 40 °C, or about 37 °C. The second temperature may be about 25 °C to about 41 °C, about 27 °C to about 41 °C, about 27 °C to about 40 °C, about 30 °C to about 40 °C, about 30 °C to about 37 °C, about 30 °C to about 35 °C, about 30 °C to about 33 °C, or about 31 °C to about 33 °C. The difference in the changed temperature may be, for example, about 1 °C to about 8 °C, about 1 °C to about 7 °C, about 1 °C to about 6 °C, about 1 °C to about 5 °C, about 1 °C to about 4 °C, about 1 °C to about 3 °C, or about 1 °C to about 2 °C.

Under the pH change condition, the cells may grow in the medium at a first pH value for about 2 days or longer, and then the pH may be changed to a second pH, which is about 0.05 to about 1 lower than the first pH value, and the cells may then grow at the second pH value for about 1 day or longer. The cells may grow in the medium at the first pH value for about 2 days or more, about 3 days or more, about 4 days or more, about 1 week or more, about 2 days to about 1 week, or about 2 days to about 4 days. The cells may be maintained in the medium at the second pH value for about 1 day or more, about 2 days or more, about 3 days or more, about 4 days or more, about 1 week or more, about 2 weeks or more, about 3 weeks or more, about 1 month or more, about 1 day to about 1 month, about 1 day or more to about 3 weeks, about 1 day or more to about 2 weeks, about 1 day or more to about 1 week, or about 1 day or more to about 4 days. The first pH value may be about pH 6.8 to about pH 7.5, about pH 6.8 to about pH 7.2, about pH 6.8 to about pH 7.0, or about pH 7.0 to about pH 7.2. The second pH value may be about pH 6.0 to about pH 7.1, about pH 6.0 to about pH 7.0, about pH 6.0 to about pH 6.8, about pH 6.0 to about pH 6.6, about pH 6.0 to about pH 6.4, or about pH 6.0 to about pH 6.2. The difference in the changed pH value may be, for example, about 0.05 to about 1, about 0.05 to about 0.9, about 0.05 to about 0.8, about 0.05 to about 0.7, about 0.05 to about 0.6, about 0.05 to about 0.5, about 0.05 to about 0.4, about 0.05 to about 0.3, about 0.05 to about 0.2, about 0.05 to about 0.1, or about 0.05 to about 0.08.

The method may further include adding glucose such that the concentration of glucose in the cell culture medium is maintained at about 2 g/L or more.

The concentration of glucose in the cell culture medium may be maintained at about 2 g/L or more, about 2.5 g/L or more, about 3.0 g/L or more, about 3.5 g/L or more, about 4 g/L or more, about 4.5 g/L or more, about 5 g/L or more, about 5.5 g/L or more, about 6 g/L or more, about 7 g/L or less, about 2 g/L to about 7 g/L, about 2 g/L to about 6 g/L, about 2.5 g/L to about 5.5 g/L, about 3 g/L to about 5 g/L, about 3.5 g/L to about 5 g/L, or about 3.5 g/L to about 4.5 g/L. The glucose may be added to the cell culture medium during cell culture.

A method of producing a target protein using a high-concentration liquid medium prepared by the above method may have improved target protein productivity, and specifically, may have improved target protein productivity compared to that achieved using a liquid medium prepared by a method including a step of adjusting the pH to hydrate the medium components and/or a step of hydrating the medium components by using a solvent at 35 °C or less.

The method may have increased expression/production level (titer) of a target protein compared to that achieved using a liquid medium prepared by a method including a step of adjusting the pH to hydrate the medium components and/or a step of hydrating the medium components by using a solvent at 35 °C or less, and specifically, the increase in expression/production level (titer) of the target protein may be about 5 % or more, about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 5 % to about 60 %, about 5 % to about 50 %, about 5 % to about 40 %, about 10 % to about 60 %, about 10 % to about 50 %, about 10 % to about 40 %, about 15 % to about 60 %, about 15 % to about 50 %, about 15 % to about 40 %, about 15 % to about 30 %, about 15 % to about 20 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 % compared to that achieved using a liquid medium prepared by a method including a step of adjusting the pH to hydrate the medium components and/or a step of hydrating the medium components by using a solvent at 35 °C or less.

The method may have increased host cell viability compared to that achieved using a liquid medium prepared by a method including a step of adjusting the pH to hydrate the medium components and/or a step of hydrating the medium components by using a solvent at 35 °C or less, and specifically, the increase in host cell viability may be about 3 % or more, about 5 % or more, about 8 % or more, about 10 % or more, about 15 % or more, about 20 % or more, about 30 % or more, about 40 % or more, about 50 % or more, about 60 % or more, about 70 % or more, about 80 % or more, about 90 % or more, about 3 % to about 50 %, about 3 % to about 40 %, about 3 % to about 30 %, about 3 % to about 20 %, about 3 % to about 10 %, about 3 % to about 5 %, about 5 % to about 50 %, about 5 % to about 40 %, about 5 % to about 30 %, about 5 % to about 20 %, about 5 % to about 10 %, about 10 % to about 15 %, about 15 % to about 20 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 % compared to that achieved using a liquid medium prepared by a method including a step of adjusting the pH to hydrate the medium components and/or a step of hydrating the medium components by using a solvent at 35 °C or less.

The method may have increased viable cell density (VCD) compared to that achieved using a liquid medium prepared by a method including a step of adjusting the pH to hydrate the medium components and/or a step of hydrating the medium components by using a solvent at 35 °C or less, and specifically, the increase in VCD may be increased by about 5 % or more, about 10 % or more, about 15 % or more, about 20 % or more, about 30 % or more, about 40 % or more, about 50 % or more, about 60 % or more, about 70 % or more, about 80 % or more, about 90 % or more, about 3 % to 5 %, about 5 % to about 10 %, about 5 % to about 60 %, about 5 % to about 50 %, about 5 % to about 40 %, about 5 % to about 30 %, about 5 % to about 20 %, about 10 % to about 60 %, about 10 % to about 50 %, about 10 % to about 40 %, about 10 % to about 30 %, about 10 % to about 20 %, about 10 % to about 15 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 % compared to that achieved using a liquid medium prepared by a method including a step of adjusting the pH to hydrate the medium components and/or a step of hydrating the medium components by using a solvent at 35 °C or less.

The method may have increased specific productivity (productivity per unit cell, qp) of host cells compared to that achieved using a liquid medium prepared by a method including a step of adjusting the pH to hydrate the medium components and/or a step of hydrating the medium components by using a solvent at 35 °C or less, and specifically, the increase in specific productivity (qp) of host cells (productivity per unit cell) may be about 10 % or more, about 15 % or more, about 20 % or more, about 30 % or more, about 40 % or more, about 50 % or more, about 60 % or more, about 70 % or more, about 80 % or more, about 90 % or more, about 99 % or more, about 100 %, about 10 % to about 60 %, about 10 % to about 50 %, about 10 % to about 40 %, about 15 % to about 60 %, about 15 % to about 50 %, about 15 % to about 40 %, about 15 % to about 30 %, about 15 % to about 20 %, about 20 % to about 30 %, about 30 % to about 40 %, about 40 % to about 50 %, about 50 % to about 60 %, about 60 % to about 70 %, about 70 % to about 80 %, about 80 % to about 90 %, or about 90 % to about 100 % compared to that achieved using a liquid medium prepared by a method including a step of adjusting the pH to hydrate the medium components and/or a step of hydrating the medium components by using a solvent at 35 °C or less.

The method may further include recovering a produced target protein from the host cells or culture medium.

Recovering the target protein or product from the host cells or culture/culture medium may be performed by any method known in the art, for example, centrifugation, filtration, chromatography, or crystallization, without being limited thereto.

This recovering process may include a purification process, which may be selected by one skilled in the art from various known purification processes, as necessary.

### Advantageous Effects of Invention

A high-concentration liquid medium, when prepared according to an aspect, may have effectively reduced osmolality. Therefore, the medium, when applied to a process for producing a target protein, can increase the production/expression level of the target protein, increase the cell count, increase the cell viability, and increase the specific productivity of the cells, thereby significantly increasing the production yield of the target protein from the cells.

### Brief Description of Drawings

FIG. 1 shows process steps of an improved method for preparing a high-concentration liquid medium.
FIG. 2 is a diagram showing a final osmolalityaccording to temperature and pH conditions in preparation of a high-concentration liquid medium.
FIG. 3 shows results of comparative analysis of viable cell density (VCD) of host cells in a target protein production process using a high-concentration liquid medium prepared by a conventional method.
FIG. 4 shows results of comparative analysis of viability of host cells in a target protein production process using a high-concentration liquid medium prepared by a conventional method.
FIG. 5 shows results of comparative analysis of the level of osmolalityin a medium in a target protein production process using a high-concentration liquid medium prepared by a conventional method.
FIG. 6 shows results of comparative analysis of expression levels (titer) of a target protein in a target protein production process using a high-concentration liquid medium prepared by a conventional method, with expression levels standardized to an expression level of D13 control.
FIG. 7 shows results of comparative analysis of viable cell density (VCD) of host cells in a target protein production process, using a high-concentration liquid medium prepared by an improved method.
FIG. 8 shows results of comparative analysis of viability of host cells in a target protein production process using a high-concentration liquid medium prepared by an improved method.
FIG. 9 shows results of comparative analysis of the level of osmolality in a medium in a target protein production process using a high-concentration liquid medium prepared by an improved method.
FIG. 10 shows results of comparative analysis of expression levels (titer) of a target protein in a target protein production process using a high-concentration liquid medium prepared by an improved method, with expression levels standardized to an expression level of D13 control.
FIG. 11 shows a more detailed comparison of expression levels (titer) of a target protein at D13 in a target protein production process using a high-concentration medium prepared by an improved method, with expression levels standardized to an expression level of D13 control.

### Best Mode for Carrying out the Invention

### Mode for the Invention

Hereinbelow, the present disclosure will be described in greater detail through experimental examples. However, these embodiments are described for illustrative purposes only and the scope of the present disclosure is not limited to these embodiments.

### Experimental Example 1: Novel Method for Preparing High-Concentration Liquid Medium

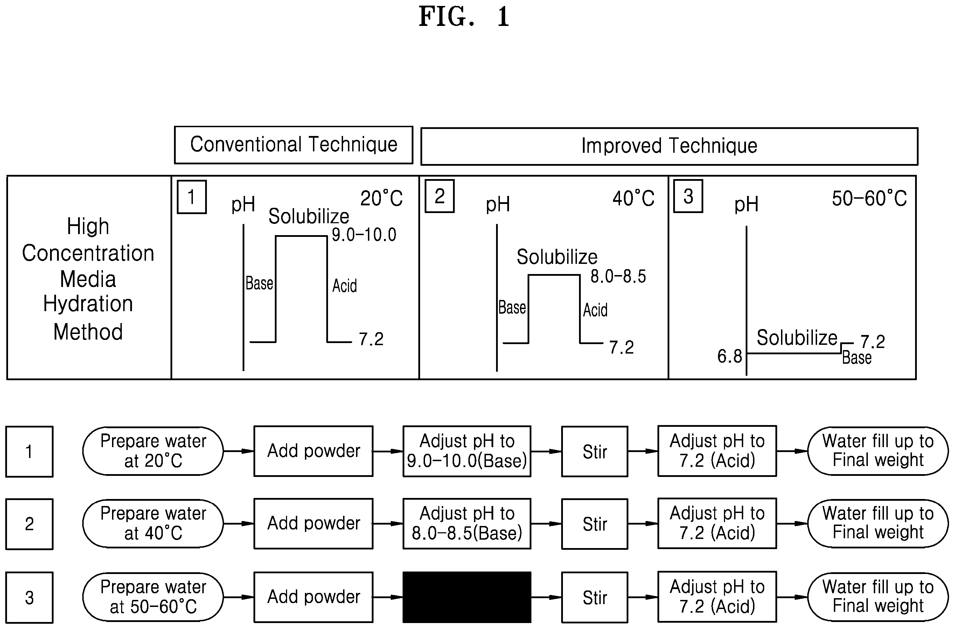

In the present disclosure, a novel method to effectively produce a high-concentration liquid medium was developed, and specifically, experiments were conducted using the following three methods (FIG. 1).

### (1) Example 1 (Control Group 1 - Conventional Technology)

① Prepared a solvent at room temperature (about 20 °C).
② Added solid medium components to the solvent.
③ Added a base to the solvent to adjust the pH to between about 9.0 and about 10.0.
④ Dissolved the added solid medium components.
⑤ Added an acid to the solution containing the dissolved solid medium components to adjust the pH to 7.2.
⑥ Added additional solvent to adjust the final concentration.

### (2) Example 2 (Experimental Group 1 - Temperature (Mild) and pH Adjustment Method)

① Prepared a solvent at about 40 °C.
② Added solid medium components to the solvent.
③ Added a base to the solvent to adjust the pH to between about 8.0 and about 8.5.
④ Dissolved the added solid medium components.
⑤ Added an acid to the solution containing the dissolved solid medium components to adjust the pH to 7.2.
⑥ Added additional solvent to adjust the final concentration.

### (3) Example 3 (Experimental Group 2 - Temperature Adjustment Method)

① Prepared a solvent at a temperature of about 50 to about 60 °C.
② Added solid medium components to the solvent.
③ Dissolved the added solid medium components.
④ Added a base to the solution containing the dissolved solid medium components to adjust the pH to 7.2.
⑤ Added additional solvent to adjust the final concentration.

In Examples 2 and 3, specifically, a high-concentration liquid medium was prepared by the following processes: ① preparing water, to be used for the preparation of a high-concentration liquid medium, at an elevated temperature of about 40 °C, or about 50 °C to about 60 °C. ② adding a main medium component, from which insulin and sodium chloride (NaCl) have been removed, to the water and carrying out hydration under stirring, and maintaining the temperature of the water at about 40 °C, or about 50 to about 60 °C during hydration. ③ when the turbidity of the hydrated solution was measured and found to be equal to or below the standard (< 3.5 NTU), cooling the solution to room temperature. ④ adding additional medium components required according to the process, and carrying out hydration. ⑤ adding an insulin stock solution. ⑥ adjusting the pH to a desired target pH, using NaOH or the like.

### Experimental Example 2: Comparative Analysis of osmolarity According to Temperature and pH Adjustment in Preparation of High-Concentration Liquid Medium

In the process of preparing a high-concentration liquid medium by hydrating (dissolving) the medium components, an experiment was conducted as described below to compare and analyze the level of osmolarity based on the temperature and pH conditions during the dissolution process.

Specifically, a medium was prepared under various temperature and pH conditions through the following process:
① preparing solvents at various temperatures (20 °C, 30 °C, 40 °C, 50 °C, and 60 °C) (the respective temperatures are maintained during the hydration process).
② adding solid medium components to the solvent, followed by mixing and dissolution.
③ if the added solid medium components are not completely dissolved, adding a base to adjust the pH, and adjusting the pH incrementally to 7.5, 8.0, 8.5, 9.0, and 9.5 until the added solid medium components are fully dissolved.
④ adding an acid or a base to the solution containing the dissolved solid medium components to adjust the pH to 7.2.
⑤ measuring the osmotic pressure of the fully dissolved medium.

In Experimental Example 2, for the experimental procedure, the main medium component and additional medium components such as insulin were separately dissolved in the same manner as in Example 2 and Example 3 of Experimental Example 1, and when the solid medium components were fully dissolved, the total amino acid concentration in the medium was 671 mM.

Meanwhile, the criterion for considering the medium components to be fully dissolved was that the NTU (nephelometric turbidity unit) measurement was 3.5 or less, with no visible particles to the naked eye. The criterion for considering the medium components not fully dissolved was that the NTU measurement exceeded 3.5 or that particles were visible to the naked eye.

In this process, the NTU value was measured using a method employing nephelometry to detect light scattering by turbidity particles, specifically, utilizing a 2100Q turbidity meter, and the detailed measurement procedure is described below.

### 1) Calibration

Calibration standard solutions (20 NTU, 100 NTU, 800 NTU) were measured to calibrate the instrument, and "Save" was pressed to complete the calibration.

### 2) Sample Measurement

The sample to be measured was dispensed into the sample cell (at least 10 mL). Before the measurement, foreign substances on the outer surface of the sample cell were removed using a cloth. Any dust remaining from the cloth was wiped away using a Kimwipe. When the sample was inserted for measurement, the triangular mark on the instrument was aligned with the arrow mark on the sample cell holder. After the "Read" button was pressed and the message "Stabilizing..." appeared on the screen, the result was displayed, indicating that measurement was complete. When the measurement was finished, the sample inside the sample cell was discarded. The inside and outside of the sample cell were then rinsed with DW for one minute each time, a total of three times. After rinsing was complete, the sample cell was filled with DW, closed with a lid, and then stored.

In addition, the osmolarity was measured using an osmometer (VWR, Advanced Osmometer 2020).

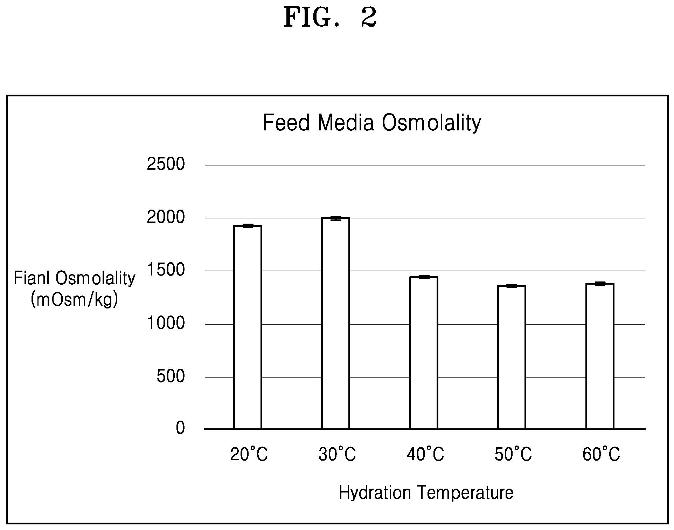

From these experimental results, it was confirmed that, when using a solvent at a lower temperature, it was necessary to adjust to a higher pH condition in order to completely dissolve the solid components. In the case of a high-concentration liquid medium prepared under high-temperature and low-pH conditions, the final osmolality was found to be significantly reduced (see FIG. 2 and Table 1). Specifically, compared to using solvents at 20 °C or 30 °C, using a solvent at 40 °C or higher resulted in a significant reduction in osmolarity and in osmolality relative to total amino acid concentration. In particular, when a solvent at 50 °C or higher was used, a significantly low osmolarity was observed even without pH adjustment.

**[Table 1]**

| **Category** | | **Hydration Temperature** | | | | |
|---|---|---|---|---|---|---|
| | | **20 °C** | **30 °C** | **40 °C** | **50 °C** | **60 °C** |
| **Hydration Target pH** | **N/A** | Insoluble Particle | Insoluble Particle | Insoluble Particle | Soluble (1.0 NTU) | Soluble (1.2 NTU) |
| | **7.5** | | | | - | - |
| | **8.0** | | | | - | - |
| | **8.5** | | | Soluble (0.8 NTU) | - | - |
| | **9.0** | | | - | - | - |
| | **9.5** | Soluble (1.1 NTU) | Soluble (1.0 NTU) | - | - | - |
| **Final Osmolality (mOsm/kg)** | | 1,936 [SD: 10] | 2,003 [SD: 11] | 1,445 [SD: 6] | 1,360 [SD: 2] | 1,383 [SD: 9] |
| Osmolality Relative to Total Amino Acid Concentration **(mOsm/kg·mM)** | | 2.89 | 2.99 | 2.15 | 2.03 | 2.06 |

Based on the results above, it can be seen that using the method of preparing a high-concentration liquid medium of the present disclosure (Experimental Group 2) enables the production of a high-concentration liquid medium with a significantly low osmolality.

### Experimental Example 3: Comparative Analysis of Osmolality and Recombinant Protein Production Efficiency of High-Concentration Liquid Media Prepared by Conventional Technique

To investigate whether using a high-concentration liquid medium prepared by a conventional liquid medium manufacturing technique (pH adjustment)-namely, the method of Example 1 (control group) in Experimental Example 1-can improve protein production efficiency and the like in a recombinant protein production process, the following experiment was performed.

Specifically, as a cell line used in the recombinant protein production process, an antibody-expressing CHO cell line was prepared by transfecting a vector expressing an antibody into a CHO cell line. This cell line was then cultured in a basal medium. After day 1 of culture, feeding of the medium was carried out, and the cells were respectively cultured in either a low-concentration medium (control, 2.0x) or a high-concentration medium (High conc. Media) (2.8x or 3.6x) prepared by the method of Example 1. In addition, the culture was performed at a bioreactor level as a fed-batch process for a total of 15 days. Based on the cell growth, the culture temperature was changed from 36 °C to 32 °C on day 3 of culture. Feeding of the medium was performed from day 1 through day 14.

The total amino acid concentrations of the 2.0x, 2.8x, and 3.6x media were 553 mM, 774 mM, and 995 mM, respectively.

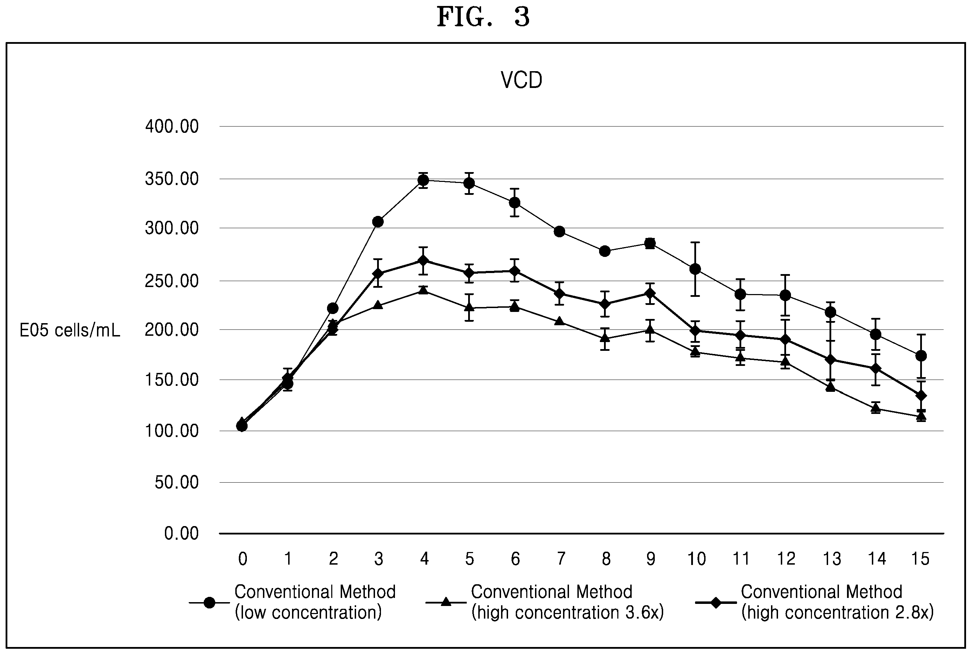

FIGS. 3 to 6 show the viable cell density (VCD), cell viability, osmolality, and recombinant protein titer in a recombinant protein production process using the medium prepared by the conventional method (Example 1) as described above.

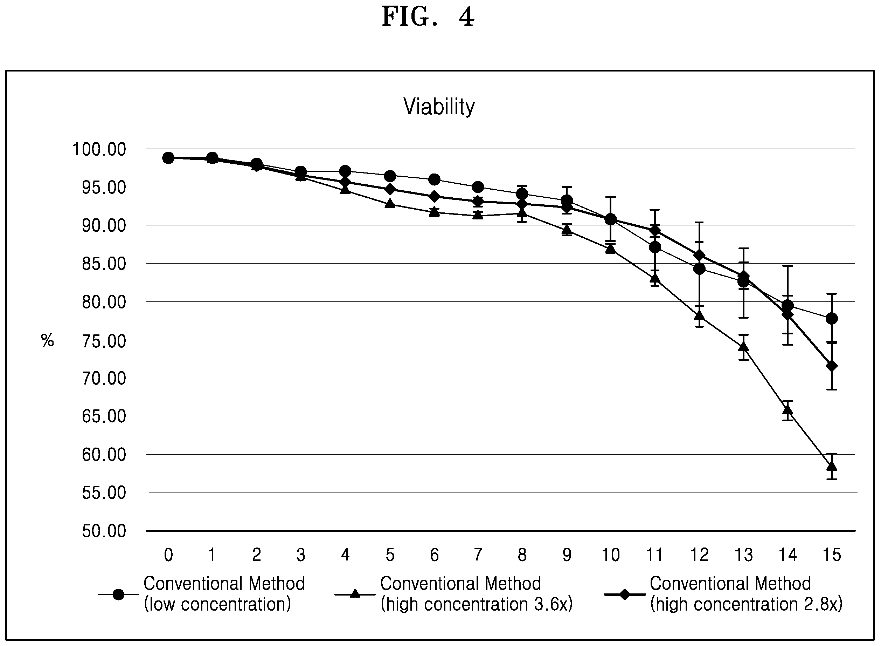

Specifically, as shown in FIGS. 3 and 4, when the high-concentration medium was used, the culture process as a whole exhibited significantly lower VCD and cell viability compared to the control group. Therefore, based on these results, even though the high-concentration medium prepared by adjusting the pH as in the conventional method was rich in nutrients, the cell viability index in the recombinant protein production process was significantly reduced.

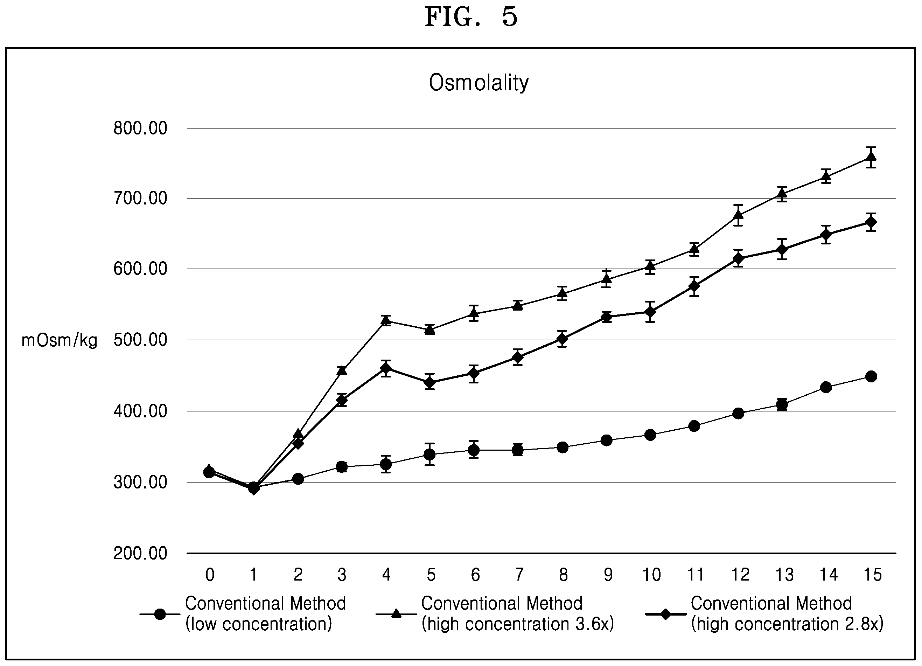

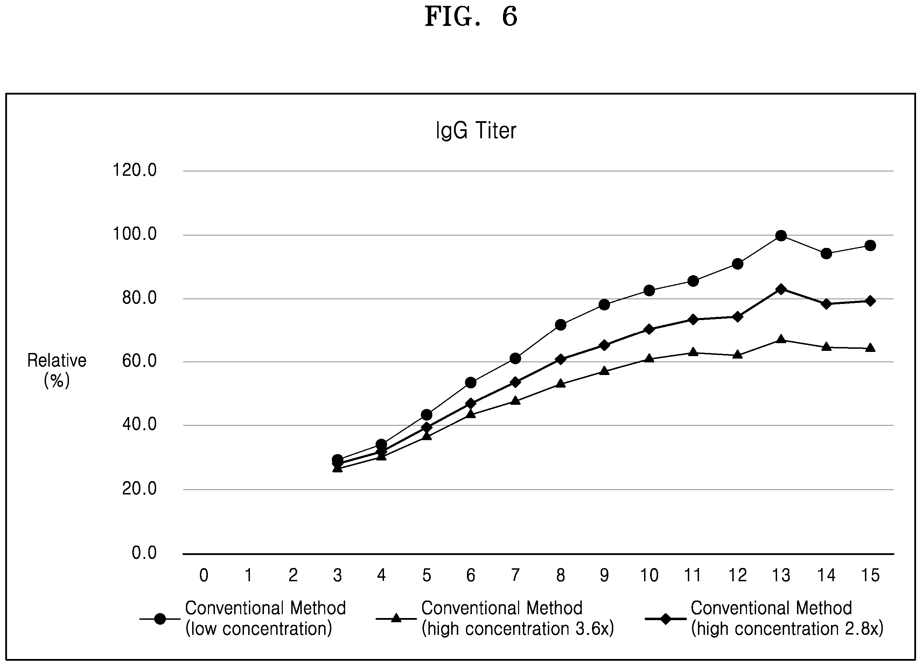

Next, as shown in FIGS. 5 and 6, in the case of the control group, the osmolality remained at a low level throughout the culture process. However, when a high-concentration medium was used, the osmolality rose significantly. Moreover, the target protein expression level (titer) was noticeably lower for higher-concentration media. Therefore, based on these results, even though the high-concentration medium prepared by adjusting the pH as in the conventional method was rich in nutrients, the productivity of recombinant protein was rather decreased.

### Experimental Example 4: Comparative Analysis of Osmolality and Recombinant Protein Production Efficiency of High-Concentration Liquid Medium Prepared Using the Novel Method

To determine whether using a high-concentration liquid medium prepared by the methods of Example 2 (Experimental Group 1) and Example 3 (Experimental Group 2) in Experimental Example 1-which correspond to the novel high-concentration liquid medium manufacturing technology (temperature control) of the present disclosure-can improve protein production efficiency in a recombinant protein production process, the following experiment was performed.

Specifically, as a cell line to be used in the recombinant protein production process, an antibody-expressing CHO cell line was prepared by transfecting a CHO cell line with a vector expressing an antibody, and the cells were cultured in a basal medium. After day 1 of culture, feeding of the medium was carried out, and the cells were respectively cultured in either a low-concentration medium (control, 2.0x, total amino acid concentration of 447 mM) prepared by the method of Example 1, a high-concentration medium (3.0x at 40 °C) prepared by the method of Example 2, or a high-concentration medium (3.0x at 50 °C) prepared by the method of Example 3. In addition, the culture was performed at a bioreactor level as a fed-batch culture for 15 days. Based on the cell growth, the culture temperature was changed from 36 °C to 32 °C on day 3 of culture. Feeding of the medium was performed from day 1 through day 14. The total amino acid concentration of the 3.0x medium was 671 mM.

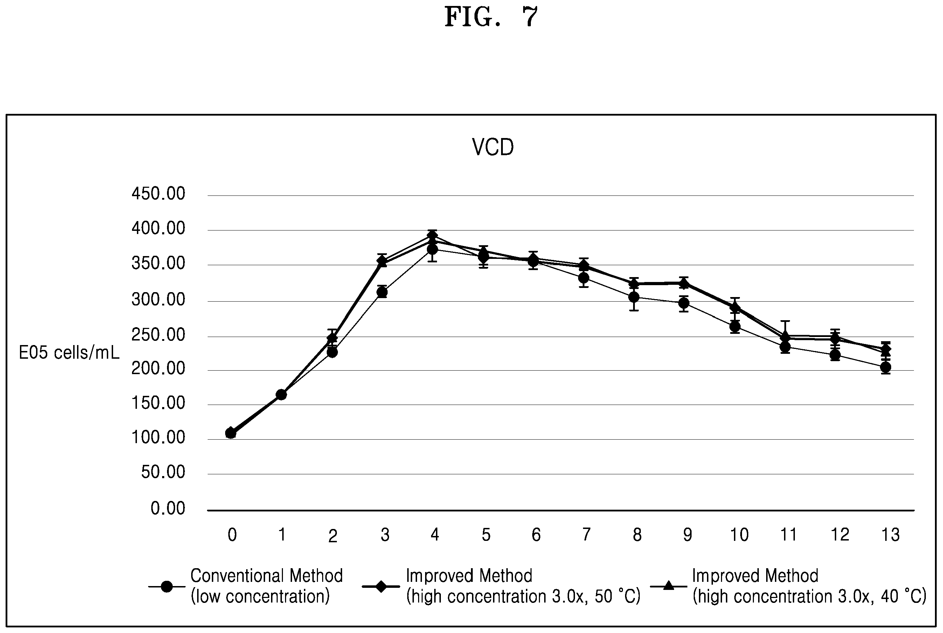

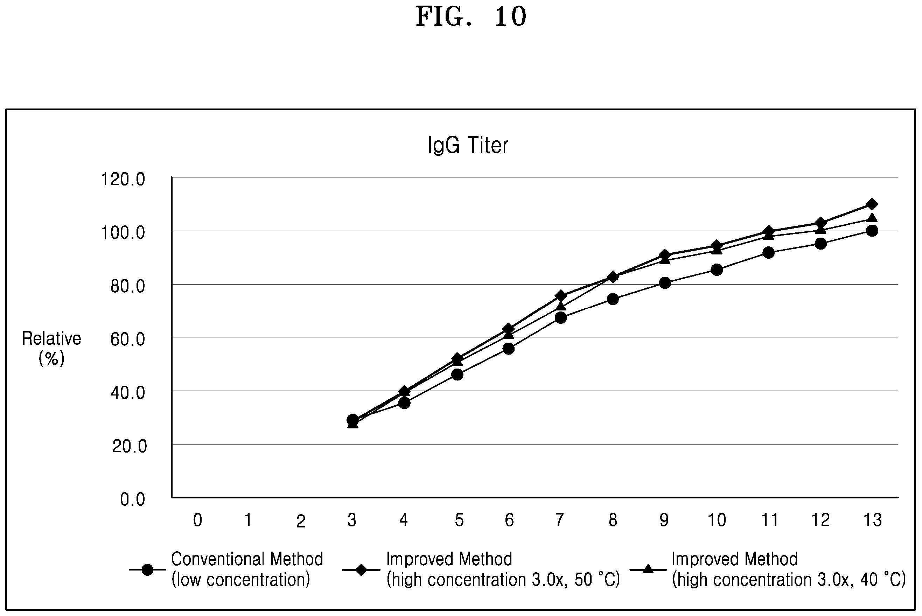

FIGS. 7 to 10 show the viable cell density (VCD), cell viability, osmolality, and recombinant protein titer in the recombinant protein production process using the high-concentration liquid media prepared by the improved method (Examples 2 and 3).

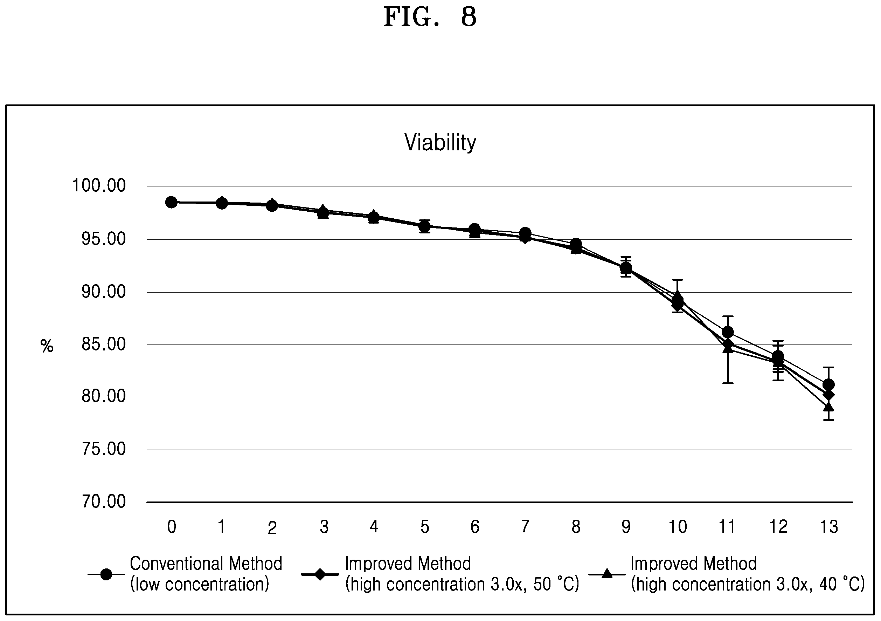

Specifically, as shown in FIGS. 7 and 8, when the high-concentration liquid medium prepared using the improved method was employed, the overall culture process exhibited a viable cell density (VCD) and cell viability similar to or higher than those of the control group. Therefore, based on these results, it can be seen that when a high-concentration medium is prepared using the improved method, cell viability indicators in the recombinant protein production process are maintained or improved compared to those obtained using the low-concentration medium prepared by the conventional method.

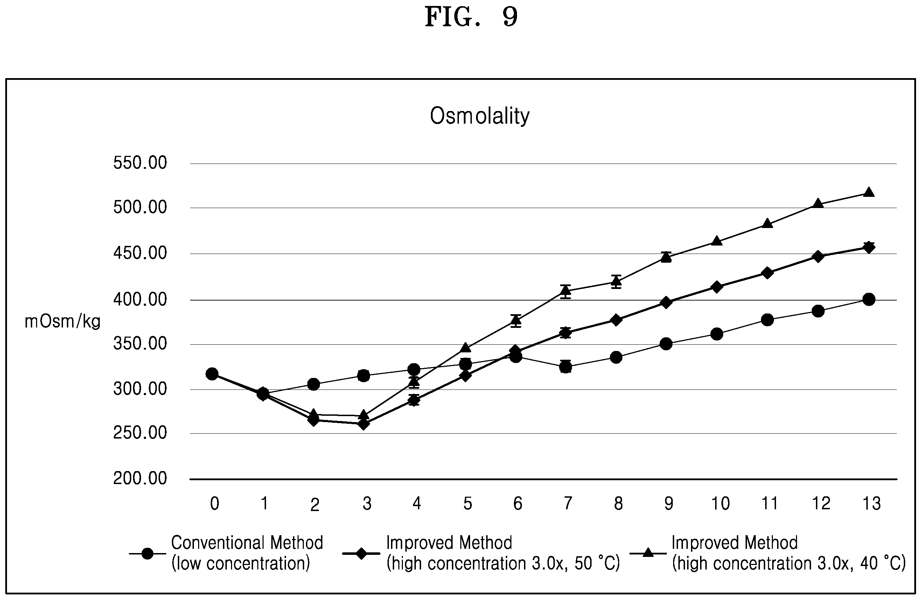

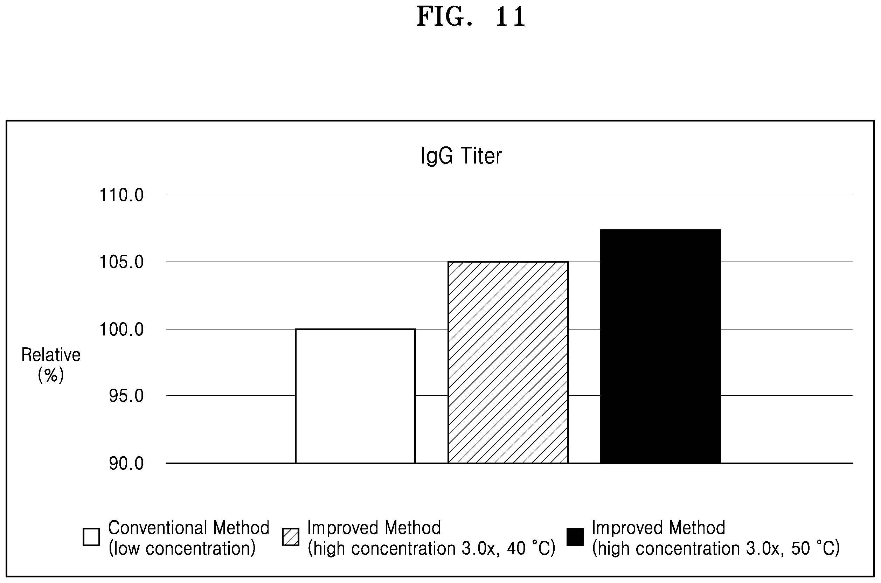

Next, as shown in FIGS. 9 to 11, the high-concentration medium prepared by the improved method, although it has a slightly higher osmolality than the control group, maintains a significantly lower osmolality compared to that of the medium prepared by the conventional method. Furthermore, it was found that in the case of the high-concentration medium prepared by the improved method, the target protein expression level (titer) increases as the concentration of the medium increases. In particular, the target protein expression level on day 13 of culture was found to increase by about 5 % or more, demonstrating a significant improvement (FIG. 11).

Therefore, based on these results, unlike the high-concentration medium prepared by the conventional method, the high-concentration medium prepared by the improved method provides a significantly improved productivity of recombinant protein.

While the present disclosure has been described with reference to exemplary embodiments, it will be apparent to those skilled in the art that various changes and modifications may be made without departing from the spirit and scope of the disclosure. Therefore, the embodiments described above should be considered in all respects as illustrative and not restrictive.

## Claims

1. A method of preparing a high-concentration liquid medium, the method comprising:
1) mixing a first medium component with a solvent at a temperature of 35 °C or more to hydrate the first medium component;
2) cooling a solution containing the hydrated first medium component; and
3) mixing a second medium component with the solution to hydrate the second medium component.

2. The method of claim 1, wherein the solvent in step 1) has a temperature of 35 °C to 100 °C.

3. The method of claim 1, wherein the solvent in step 1) has a temperature of 40 °C to 80 °C.

4. The method of claim 1, wherein the solvent in step 1) has a temperature of 50 °C to 80 °C.

5. The method of claim 1, wherein the solvent is water.

6. The method of claim 1, wherein the first medium component comprises a component that does not undergo denaturation at a temperature of 35 °C or more.

7. The method of claim 1, wherein the first medium component does not comprise a component capable of being denatured at a temperature of 35 °C or more.

8. The method of claim 1, wherein step 1) is performeduntil a turbidity of the solution containing the hydrated first medium component reaches 10 NTU or less.

9. The method of claim 1, wherein the solution in step 2) is cooled to a temperature of 10 °C to 30 °C.

10. The method of claim 1, wherein the second medium component comprises a component capable of being denatured at a temperature of 35 °C or more.

11. The method of claim 1, wherein the first medium component and the second medium component do not comprise sodium chloride.

12. The method of claim 1, wherein step 3) is performed until a turbidity of the solution containing the hydrated second medium component reaches 10 NTU or less.

13. The method of claim 1, further comprising:
4) mixing an acid or a base with the solution containing the hydrated second medium component, to adjust pH.

14. The method of claim 13, wherein step 4) comprises adjusting pH of the solution to a range of 6.5 to 7.5.

15. The method of claim 1, wherein the method does not comprise adjusting the pH with an acid or a base during the hydration of the first medium component or the second medium component.

16. The method of claim 1, wherein, when a temperature of the solvent used in step 1) is 35 °C to 45 °C, the method comprises adjusting pH with an acid or a base in the step of hydration of the first medium component and/or the second medium component.

17. The method of claim 16, wherein the adjustment of the pH comprises using a base to adjust pH to between 7.5 and 9.0.

18. The method of claim 1, wherein the method is for producing a high-concentration liquid medium with reduced osmolarity.

19. The method of claim 1, wherein the method is for producing a liquid medium having an osmolarity of 500 mOsm/kg to 3,000 mOsm/kg.

20. The method of claim 1, wherein osmolarity of a liquid medium prepared by the method is decreased compared to that of a liquid medium prepared by a method including a step of adjusting pH to hydrate medium components and/or a step of hydrating medium components by using a solvent at 35 °C or less.

21. The method of claim 1, wherein a liquid medium prepared by the method has an osmolarity decreased by about 5 % or more as compared to that of a liquid medium prepared by a method including a step of adjusting pH to hydrate medium components and/or a step of hydrating medium components by using a solvent at 35 °C or less.

22. The method of claim 1, wherein the method is for producing a liquid medium comprising total amino acids at a concentration of 300 mM to 1,500 mM.

23. The method of claim 1, wherein the method is for producing a liquid medium having an osmolality relative to total amino acid concentration of 0.5 mOsm/kg·mM to 3.0 mOsm/kg·mM.

24. The method of claim 1, wherein osmolality relative to total amino acid concentration (mOsm/kg·mM) of a liquid medium prepared by the method is decreased by about 5 % or more as compared to that of a liquid medium prepared by a method including a step of adjusting pH to hydrate medium components and/or a step of hydrating medium components by using a solvent at 35 °C or less.

25. The method of claim 1, wherein the method is for producing a high-concentration liquid medium having improved target protein productivity.

26. The method of claim 25, wherein the improved target protein productivity comprises one or more of the following characteristics:
1) improved viability of host cells that express or produce a target protein;
2) improved viable cell density (VCD) of host cells in a target protein production process; and
3) improved expression/production level (titer) of a target protein in a target protein production process.

27. The method of claim 1, wherein the method comprises:
1) mixing a first medium component with a solvent at a temperature of 35 °C to 45 °C to hydrate the first medium component;
2) cooling a solution containing the hydrated first medium component to room temperature;
3) mixing a second medium component with the solution to hydrate the second medium component; and
4) adjusting pH of the solution to between 7.0 and 7.5,
wherein step 1) and/or step 3) comprises adjusting pH of the solvent/solution to between 7.5 and 8.5 to hydrate the medium components.

28. The method of claim 1, wherein the method comprises:
1) mixing a first medium component with a solvent at a temperature of 45 °C to 65 °C to hydrate the first medium component;
2) cooling a solution containing the hydrated first medium component to room temperature;
3) mixing a second medium component with the solution to hydrate the second medium component; and
4) adjusting pH of the solution to between 7.0 and 7.5.

29. A high-concentration liquid medium prepared by any one of claims 1 to 28.

30. A method of producing a target protein, the method comprising culturing a host cell expressing a target protein by using a high-concentration liquid medium prepared by the method of any one of claims 1 to 28.

31. The method of claim 30, wherein an expression/production level (titer) of the target protein is increased by about 5 % or more as compared to that obtained by a method using a liquid medium prepared by a method including a step of adjusting pH to hydrate medium components and/or a step of hydrating medium components by using a solvent at 35 °C or less.

32. The method of claim 30, wherein viability of the host cell is increased by about 3 % or more as compared to that obtained by a method using a liquid medium prepared by a method including a step of adjusting pH to hydrate medium components and/or a step of hydrating medium components by using a solvent at 35 °C or less.

33. The method of claim 30, wherein viable cell density (VCD) of the host cell is increased by about 5 % or more as compared to that obtained by a method using a liquid medium prepared by a method including a step of adjusting pH to hydrate medium components and/or a step of hydrating medium components by using a solvent at 35 °C or less.
